(19) Europäisches Patentamt / European Patent Office / Office européen des brevets

(11) **EP 3 934 527 B1**

(12) **EUROPEAN PATENT SPECIFICATION**

(45) Date of publication and mention
of the grant of the patent:
**06.05.2026 Bulletin 2026/19**

(21) Application number: **20770991.6**

(22) Date of filing: **06.03.2020**

(51) International Patent Classification (IPC):
**A61B 5/024** (2006.01)      **A61B 5/333** (2021.01)

(52) Cooperative Patent Classification (CPC):
**A61B 5/02405; A61B 5/0006; A61B 5/02455;
A61B 5/282; A61B 5/304; A61B 5/327;
A61B 5/332; A61B 5/333; A61B 5/352;
A61B 5/681; A61B 5/6824; A61B 5/7214;
A61B 5/742; A61B 5/746;** A61B 2560/0468;
(Cont.)

(86) International application number:
**PCT/CA2020/050304**

(87) International publication number:
**WO 2020/181367 (17.09.2020 Gazette 2020/38)**

(54) **LEADLESS ELECTROCARDIOGRAM MONITOR**

ELEKTRODENLOSER ELEKTROKARDIOGRAMM-MONITOR

MONITEUR D'ÉLECTROCARDIOGRAMME SANS FIL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.03.2019 CA 3036168
14.03.2019 US 201916353894
17.07.2019 US 201916514571
17.07.2019 US 201916514727**

(43) Date of publication of application:
**12.01.2022 Bulletin 2022/02**

(73) Proprietor: **The Access Technologies
Ottawa, Ontario K2H 5Z3 (CA)**

(72) Inventors:
• **AHMAD, Saif
Kanata, Ontario K2M 2L6 (CA)**
• **GARG, Atul K.
Kanata, Ontario K2W 1A5 (CA)**

(74) Representative: **SONN Patentanwälte GmbH & Co
KG
Riemergasse 14
1010 Wien (AT)**

(56) References cited:
EP-A1- 2 842 483      EP-A1- 3 242 587
WO-A1-2018/170607      WO-A1-2019/042486
US-A1- 2012 022 341      US-A1- 2016 317 067
US-A1- 2017 127 966      US-A1- 2018 250 510
US-B2- 7 894 888      US-B2- 9 008 761

(52) Cooperative Patent Classification (CPC): (Cont.)
A61B 2562/04

**Description**

## TECHNICAL FIELD

In general, this invention relates to electrocardiogram (ECG) monitoring in humans with wearable technology, and in particular to continuous and unobtrusive ECG monitoring utilizing a pair of ergonomically designed wireless smart bands that the user wears around the left and right wrists.

## BACKGROUND

A regular ECG test is an essential diagnostic tool that characterizes the heart's activity at a given point in time. Abnormal heart rhythms and cardiac symptoms may however sporadically appear, disappear, and reappear over time. Consequently, point-in-time ECG tests may miss critical cardiac anomalies, thereby leading to an increased risk of morbidity and mortality.

It is therefore important to monitor ECG continuously in at-risk patients as they go about their normal activities. Quite often, serious heart conditions like atrial fibrillation (AF), cardiomyopathy, and coronary heart disease are diagnosed with continuous ECG monitoring. This allows for timely clinical interventions like medication and cardiac surgery that reduce adverse outcomes like stroke and heart attack, thereby saving lives.

In clinical practice, it is common to undertake continuous ECG monitoring using a Holter system that can generally record 24-48 hours of cardiac data. The Holter is a small wearable biopotential measurement device comprising several ECG leads. These ECG leads are snapped on to sticky gel electrodes that are attached at various locations on the patient's chest. A Holter monitoring system is inconvenient and obtrusive due to the sticky gel chest electrodes that often cause discomfort and the unwieldy leads that hang between the electrodes and the Holter unit.

Recently, Medtronic has developed and marketed a leadless Holter system (SEEQ™) in the form of an adhesive chest strip (~ 4.5" long, ~ 2.0" wide, and ~ 0.6" thick) for continuous ECG monitoring.

Though leadless, this monitor is awkward and uncomfortable because it uses sticky chest electrodes and it is too bulky to be attached to the chest.

Various kinds of belts that can be worn around the chest for continuous ECG monitoring are available in the market today. Many of these ECG chest belt systems are leadless and employ dry reusable electrodes. Still, these ECG belts need to be worn under clothing and are often quite tight around the chest, causing difficulty and uneasiness to the wearer.

Currently, continuous ECG monitoring technology comes with a number of problems and encumbrances. These include discomfort, uneasiness, sleep disruptions, difficulty in carrying out day-to-day activities, and inability to undertake long-term monitoring (for example, monitoring for days, months, and years).

With the advent of newer generation wearables like smartwatches, attempts have been made to integrate ECG monitoring into a smartwatch. For example, Apple has provided dry ECG electrodes on the backplate of a smartwatch (left-side electrodes) and a second set of electrodes on the smartwatch rim (right-side electrodes). A user has to wear the smartwatch on one wrist so that the electrodes underneath touch the wrist. Additionally, the user has to touch the second set of electrodes on the smartwatch rim with his/her other hand so that the heart lies in-between the left-side (backplate) and right-side (rim) electrodes that are electrically connected to signal amplification/conditioning circuitry inside the smartwatch. The quality of ECG signal acquired in this manner is generally satisfactory. However, the main limitation is that the user has to touch and hold a second set of electrodes on the smartwatch with his/her other hand for monitoring ECG waveform data. As a result, this system only provides an on demand 30 seconds of ECG monitoring, and not continuous and/or long-term ECG monitoring.

To avoid touching a second set of electrodes with the other hand and to accomplish leadless continuous ECG monitoring, attempts have been made to develop wearable single upper limb ECG systems.

Prior art has proposed the use of single arm wearable devices for leadless ECG monitoring. These systems comprise an upper arm band with more than one electrode on the underside that come in contact with the arm when the band is worn. The electrodes are interfaced with an amplification and control unit that may be affixed to the outer surface of the band. Single arm ECG systems have produced mixed results for a diverse population. The ECG signal acquired by these systems is often noisy, unreliable, and unusable, more so for women and older people.

Based on the principles of single arm ECG systems, other prior art has also proposed leadless ECG monitoring employing wearable single wrist systems. The quality and fidelity of data acquired by single wrist ECG systems has not been properly tested and/or verified. Intuitively, a single wrist ECG system will produce noisier and weaker signals as compared to a single arm ECG system. This is because the wrist is physically farther away from the heart as compared to the upper arm, thus resulting in greater impedance to the flow of electrical charge from the heart to the wrist electrodes.

## SUMMARY

The invention is defined by the appended claims. In one aspect of the present disclosure there is disclosed a wearable device related to ECG monitoring technology. The wearable device comprises a pair of ergonomically designed wireless smart bands that are worn around the left and right wrists for unobtrusive continuous leadless ECG waveform data monitoring and analysis. Both smart bands in the described pair are provided with dry reusable ECG electrodes on their underside. The electrodes in each smart band are interfaced with biopotential measurement hardware and software inside that smart band. Moreover, the hardware and software inside the two smart bands enables seamless wireless communication between them. When the two smart bands are worn on both hands, their respective electrodes come in contact with the left and right side of the body. With this configuration, the two smart bands independently measure biopotential on the left and right side of the body and wirelessly share/process this information to acquire/analyze high-fidelity ECG waveform data. Thus, the wireless smart band pair accomplishes ECG waveform data monitoring and analysis as per Einthoven's law without the need for physically completing a circuit via leads and/or touching and holding auxiliary electrodes. In one example, the two smart bands independently and simultaneously measure biopotential on the left and right side of the body.

The two smart bands in the described pair are alluded to as a primary smart band and a secondary smart band. Both the primary and secondary smart bands preferably comprise electrodes, ECG amplification/conditioning circuitry, a microcontroller, a wireless transceiver, and a rechargeable battery. The primary smart band can be additionally provided with memory and a touchscreen display. Both the primary and secondary smart bands preferably have wireless charging capabilities and can be charged on a twin wireless charging unit.

In one embodiment, both primary and secondary smart bands are provided with three ECG strip electrodes on their underside to maximize the electrode surface area and enhance connectivity around the wrist to obtain high-quality ECG signal. Each of the three strip electrodes can be arranged to have a rigid section on the smart band backplate and a flexible section along the underside of the smart band straps. In one example, the rigid electrodes are made of silver while the flexible electrodes are made of conductive fabric.

In one example, in both smart bands, the first strip is a right-side electrode and the second strip is a left-side electrode connected to a biopotential amplifier while the third strip is a ground electrode. In another example, in both smart bands, the right-side and left-side strip electrodes remain unchanged while the third strip is a right leg drive (RLD) electrode to reduce common mode noise and augment ECG signal quality. In yet another example, in both smart bands, the right-side and left-side strip electrodes remain unchanged while the third strip in the secondary smart band is a ground electrode and the third strip in the primary smart band is an RLD electrode for enhancing ECG waveform data quality.

In one example, the primary smart band is worn around the left wrist while the secondary smart band is worn around the right wrist. With this setup, the primary smart band acquires biopotential data from the left side of the body. Simultaneously, the secondary smart band acquires biopotential data from the right side of the body and transmits this information wirelessly to the primary smart band. Biopotential information from the left and right side of the body is processed and combined inside the primary smart band using a variety of methods to acquire high-fidelity ECG signal.

In one example, the biopotential data from both smart bands is sent directly to the microcontroller inside the primary smart band for processing and obtaining an ECG signal. In another example, the biopotential data from both smart bands is first sent to a differential amplifier for further amplification/conditioning and then to the microcontroller inside the primary smart band for processing and obtaining an ECG signal.

In a further embodiment, inside each smart band, a digital switch can be provided between each of the three strip electrodes and the associated signal amplification/conditioning circuitry, resulting in three digital electrode switches inside each smart band. These electrode switches allow various electrode configurations to be evaluated for enhancing ECG waveform data quality. This feature is useful for device testing and calibration whereby an optimum electrode configuration that results in best ECG signal quality can be readily determined. In one example, all three electrodes, namely, right, left, and RLD of the primary smart band are switched on while only right and left electrodes of the secondary smart band are switched on.

In a further aspect, the microcontroller inside the primary smart band analyzes the acquired ECG waveform data in real-time to compute parameters like heart rate (HR) and heart rate variability (HRV) and to generate alerts when these parameters are out of range. For example, if HRV is above a given threshold, an AF alert is generated. The primary smart band displays real-time ECG waveform data along with metrics like HR and HRV and any alerts that are generated. The onboard memory in the primary smart band stores all ECG-related information. The primary smart band can also have the functionality to send the acquired ECG waveform data and related information wirelessly to a smartphone, personal computer (PC), tablet, or directly to a cloud server where it can be further processed/analyzed.

Fusion of DSP and Analog Signal Conditioning Techniques

In a further embodiment employing fusion of DSP and analog signal conditioning techniques, in both smart bands, the first strip is a right-side electrode and the second strip is a left-side electrode connected to a biopotential amplifier while the third strip is a reference electrode. In another example, in both smart bands, the right-side and left-side strip electrodes remain unchanged while the third strip or the reference electrode is a ground electrode. In yet another example, in both smart bands, the right-side and left-side strip electrodes remain unchanged while the third strip or the reference electrode is a right leg drive (RLD) electrode to reduce common mode noise and augment ECG signal quality. Finally, in another example, in both smart bands, the right-side and left-side strip electrodes remain unchanged while the third strip or reference electrode in the secondary smart band is a ground electrode and the third strip or reference electrode in the primary smart band is an RLD electrode for enhancing ECG waveform data quality.

In one embodiment, inside each smart band, a digital switch is provided between each of the three strip electrodes and the associated signal amplification/conditioning circuitry, resulting in three digital electrode switches inside each smart band. In another embodiment, the digital switch of the third strip or reference electrode in each smart band is a changeover switch that is used to convert the reference electrode into either a ground or RLD electrode. In one example, all digital switches inside the two smart bands are controlled by the respective microcontrollers inside the smart bands. These electrode switches allow various electrode configurations to be evaluated and used for enhancing ECG waveform data quality. This feature is useful for device testing and calibration whereby an optimum electrode configuration that results in best ECG signal quality can be readily determined and employed. In an example configuration, all three electrodes, namely, right, left, and RLD of the primary smart band are enabled while only right and left electrodes of the secondary smart band are enabled.

In one example, the biopotential data from both smart bands is sent directly to the microcontroller inside the primary smart band whereby various digital signal processing (DSP) techniques are employed to obtain an ECG signal. In another example, the biopotential data from both smart bands is first sent to a differential amplifier for analog signal amplification and conditioning, and then to the microcontroller inside the primary smart band for processing and obtaining an ECG signal. In yet another example, the ECG information obtained via the DSP and analog signal amplification/conditioning techniques is fused by the microcontroller inside the primary smart band to obtain an ECG signal of even higher quality and fidelity.

Electrode Switching

Factors like individual physiology, electrode contact area, mechanical pressure on electrodes, and electrode vibration may introduce noise in the left and right side biopotential data acquired by the smart band pair. Therefore, the resulting ECG signal may also be contaminated with noise. In such scenarios, the digital switches that allow different configurations of the six electrodes of the two smart bands to be used during data acquisition can be employed to reduce noise for obtaining a high-fidelity ECG signal. However, for a given operating condition (for example, mechanical pressure on the electrodes), a particular electrode configuration may give best results whereas for another operating condition (for example, electrode vibration), a different electrode configuration may produce better results. To overcome this limitation, in one embodiment, rapid switching of the six digital switches of the two smart bands is undertaken such that left and right side biopotential data is acquired and combined every data sampling period as the system continuously and repeatedly loops through all possible electrode configurations to minimize noise and enhance signal quality under all operating conditions.

In one example using electrode switching, the biopotential data from both smart bands is sent directly to the microcontroller inside the primary smart band whereby various digital signal processing (DSP) techniques are employed to obtain an ECG signal. In another example, the biopotential data from both smart bands is first sent to a differential amplifier for analog signal amplification and conditioning, and then to the microcontroller inside the primary smart band for processing and obtaining an ECG signal. In yet another example, the ECG information obtained via the DSP and analog signal amplification/conditioning techniques is fused by the microcontroller inside the primary smart band to obtain an ECG signal of even higher quality and fidelity.

Though this disclosure is described as related to a pair of wearable smart bands that are attached to a user's left and right wrists, the underlying design and principle of the disclosure can be extended to a pair of wearables that can be attached at any location along the two upper limbs and/or even the two lower limbs. One example comprises a primary smart band worn around the wrist and a secondary smart band worn around the upper arm of the other hand. Another example comprises both primary and secondary smart bands worn around the two upper arms. Yet another example comprises a primary smart band worn around the wrist and a secondary smart band worn around the ankle of the other leg. It will be appreciated that the smart band could be a smartwatch or any other similar wearable.

This disclosure fulfills the theoretical underpinnings of electrocardiography and Einthoven's law such that biopotential is measured on the left and right sides of the body with the heart in-between utilizing a pair of wirelessly synced wearables (for example, smart bands, smartwatches, and/or any combination thereof) that process all information to acquire high-fidelity single-lead ECG waveform data.

## BRIEF DESCRIPTION OF THE DRAWINGS

FIG. 1 illustrates an exemplary attachment of the wireless smart band pair on a user for continuous leadless ECG monitoring along with external devices to which data is wirelessly transmitted.

FIGS. **2A-2**C illustrate the front, side, and back of the primary smart band showing the touchscreen display along with the rigid/flexible strip electrodes and clasping studs/holes.

FIGS. **3**A-3C illustrate the front, side, and back of the secondary smart band showing the front cover along with the rigid/flexible strip electrodes and clasping studs/holes.

FIG. **4** illustrates an alternate view of the primary smart band showing the touchscreen display along with the straps, clasping mechanism, and flexible strip electrodes.

FIG. **5** illustrates an alternate view of the secondary smart band showing the front cover along with the straps, clasping mechanism, and flexible strip electrodes.

FIG. **6** illustrates an exploded view of the primary smart band showing the key components.

FIG. **7** illustrates an exploded view of the secondary smart band showing the key components.

FIG. **8** illustrates the smart band pair being charged on a twin wireless charging unit.

FIG. **9** illustrates an operational diagram of the smart band pair whereby one electrode in each is a ground and biopotential data from both smart bands is sent directly to the microcontroller for processing.

FIG. **10** illustrates an operational diagram of the smart band pair whereby one electrode in each is an RLD and biopotential data from both smart bands is sent directly to the microcontroller for processing.

FIG. **11** illustrates an operational diagram of the smart band pair whereby one electrode in each is a ground and biopotential data from both smart bands is first sent to a differential amplifier and then to the microcontroller for processing.

FIG. **12** illustrates an operational diagram of the smart band pair whereby one electrode in each is an RLD and biopotential data from both smart bands is first sent to a differential amplifier and then to the microcontroller for processing.

FIG. **13** illustrates an operational diagram of the smart band pair whereby one electrode in the secondary is a ground while one electrode in the primary is an RLD and biopotential data from both smart bands is sent directly to the microcontroller for processing.

FIG. **14** illustrates an operational diagram of the smart band pair whereby one electrode in the secondary is a ground while one electrode in the primary is an RLD and biopotential data from both smart bands is first sent to a differential amplifier and then to the microcontroller for processing.

FIG. **15** illustrates the circuit diagram of a biopotential amplifier with a ground/RLD strip electrode implemented using Analog Devices AD8232 chip.

FIG. **16** illustrates a flowchart depicting the method of continuous ECG monitoring and HR/HRV analysis whereby biopotential data from both smart bands is sent directly to the microcontroller for processing.

FIG. **17** illustrates a flowchart depicting the method of continuous ECG monitoring and HR/HRV analysis whereby biopotential data from both smart bands is first sent to a differential amplifier and then to the microcontroller for processing.

FIG. **18** illustrates examples of various locations on the human body where wearables employing the underlying design and principle of the current invention can be attached to undertake continuous leadless ECG monitoring.

FIG. **19** illustrates an example operational diagram of the smart band pair in a further embodiment using fusion of DSP and analog signal conditioning techniques.

FIG. **20** illustrates fusion of two complementary techniques, namely, DSP and analog signal conditioning to obtain a high-fidelity ECG signal.

FIG. **21** illustrates a flowchart depicting one example method of continuous high-fidelity ECG monitoring and HR/HRV analysis via fusion of two complementary techniques, namely, DSP and analog signal conditioning.

FIG. **22** illustrates an example operational diagram of the smart band pair for leadless ECG monitoring via electrode switching.

FIGS. **23A-23B** illustrates a truth table enlisting all possible states of the six electrode switches for electrode switching.

FIG. **24** illustrates a flowchart depicting one example method of continuous high-fidelity ECG monitoring and HR/HRV analysis via electrode switching.

## DETAILED DESCRIPTION

A preferred embodiment of the present disclosure will be set forth in detail with reference to the drawings, in which like reference numerals refer to like elements or method steps throughout.

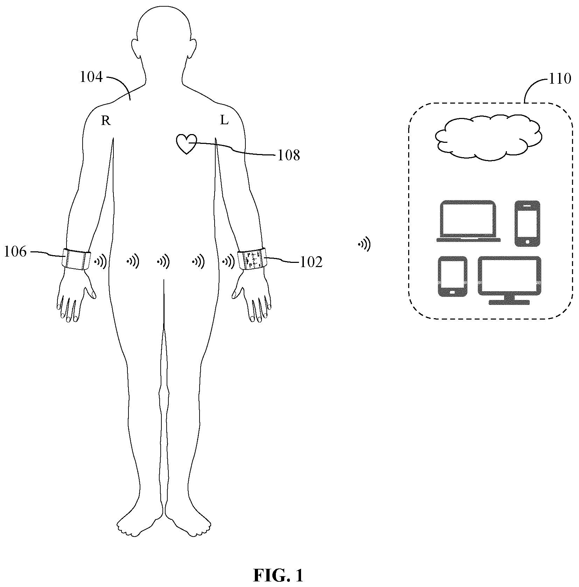

FIG. 1 illustrates an exemplary attachment of the wireless smart band pair on a user for continuous leadless ECG monitoring along with external devices to which data is wirelessly transmitted. In this example, the primary smart band **102** is worn by the user **104** around the left wrist whereas the secondary smart band **106** is worn around the right wrist. The heart **108** is shown inside the chest cavity positioned slightly towards the left. The secondary smart band **106** worn around the right wrist measures the right-side biopotential by virtue of the electrodes provided on its underside (not shown) and sends this information wirelessly to the primary smart band **102** worn around the left wrist. Simultaneously, the primary smart band **102** worn around the left wrist measures the left-side biopotential by virtue of the electrodes provided on its underside (not shown) and combines/processes this information with the wirelessly received right-side biopotential information to acquire high-fidelity ECG waveform data. The primary smart band **102** analyzes the acquired ECG waveform data, stores all information locally, and also transmits this information wirelessly to remote devices **110** like smartphones, laptops, tablets, and cloud databases for storage and further analysis. The primary **102** and secondary **106** smart bands can also be swapped between the two hands to acquire ECG waveform data in a manner similar to the one described above. That is, the primary smart band **102** can be also worn around the right wrist and the secondary smart band **106** can also be worn around the left wrist for continuous leadless ECG monitoring as outlined in the invention as defined by the appended claims.

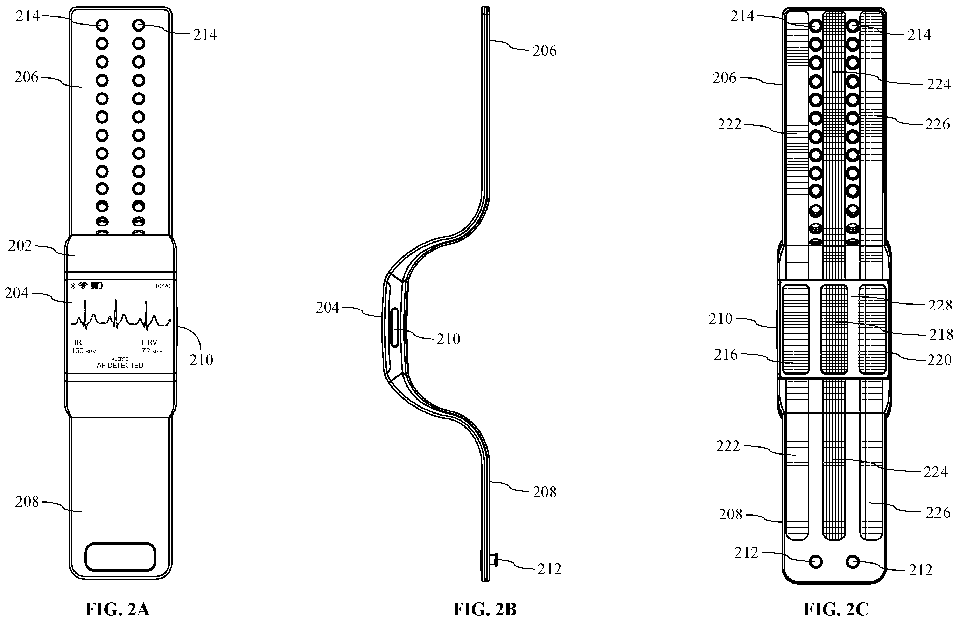

FIGS. **2A-2C** illustrate one embodiment of the front, side, and back of the primary smart band showing the touchscreen display along with the rigid/flexible strip electrodes and clasping studs/holes. The primary smart band comprises an enclosure **202** made of stainless steel, a touchscreen display **204,** upper **206** and lower **208** straps made of flexible rubber, and an on/off button **210**. Studs **212** made of hard rubber and corresponding holes **214** are provided on the primary smart band straps for clasping it snugly around the wrist. It will be appreciated that while two sets of studs **212** and holes **214** are shown, a single set, multiple sets or other arrangements could be used instead. It will also be appreciated that the display 204 could be a plain display that is not a touchscreen.

Three rigid strip electrodes **216, 218, 220** and three flexible strip electrodes **222, 224, 226** are provided on the underside of the primary smart band. The three rigid strip electrodes **216, 218, 220** are embedded in the primary smart band backplate **228** that is made of plastic. The three flexible strip electrodes **222, 224, 226** are embedded in the upper **206** and lower **208** straps of the smart band. Each of the three rigid **216, 218, 220** and flexible **222, 224, 226** strip electrodes are electrically connected inside the primary smart band. That is **rigid strip electrode 216** is connected to **flexible strip electrode 222, rigid strip electrode 218** is connected to **flexible strip electrode 224,** and **rigid strip electrode 220** is connected to **flexible strip electrode 226.** In one example, the rigid strip electrodes **216, 218, 220** are made of silver while the flexible strip electrodes **222, 224, 226** are made of silver foil. In another example, the rigid strip electrodes **216, 218, 220** are made of chrome-plated steel while the flexible strip electrodes **222, 224, 226** are made of conductive fabric. A variety of conductive materials can be used to fabricate the rigid and flexible strip electrodes described in this invention as defined by the appended claims.

In one example, the approximate dimensions of the primary smart band enclosure **202** are 43.0 mm (length) x 42.0 mm (width) x 9.5 mm (height). The width of the straps **206, 208** is approximately 41.0 mm and closely matches the length of the smart band enclosure **202.** The approximate width of the rigid **216, 218, 220** and flexible **222, 224, 226** strip electrodes is 8.5 mm and the approximate separation between them is 5.5 mm. In this example, the 5.5 mm gap between the flexible strip electrodes **222, 224, 226** conveniently allows for the primary smart band clasping studs **212** and holes **214** to be provided within this gap. The approximate weight of such a primary smart band is 40 g.

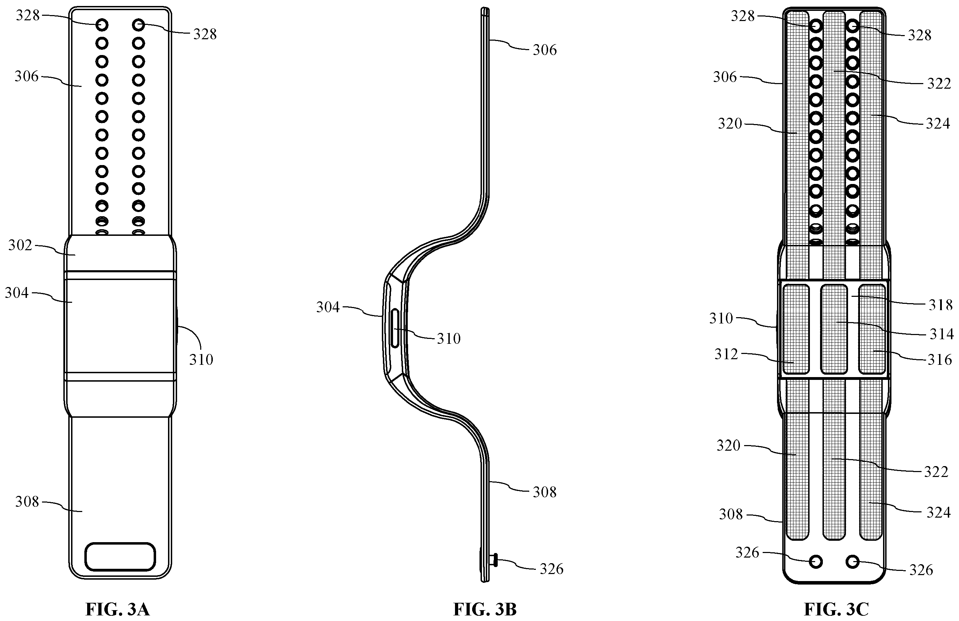

FIGS. **3**A-**3**C illustrate one embodiment of the front, side, and back of the secondary smart band showing the front cover along with the rigid/flexible strip electrodes and clasping studs/holes. The design, footprint, materials, dimensions, weight, and fabrication of the secondary smart band is similar to that of the primary smart band. The only difference is that the secondary smart band does not have a display. In this example, the secondary smart band comprises an enclosure **302** made of stainless steel, a plastic front cover **304,** upper **306** and lower **308** straps made of flexible rubber, and an on/off button **310.** It also comprises three rigid strip electrodes **312, 314, 316** embedded in a plastic backplate **318** and three flexible strip electrodes **320, 322, 324** embedded in the upper **306** and lower **308** straps. Studs **326** made of hard rubber and holes **328** are provided on the secondary smart band straps for clasping it snugly around the wrist. It will be appreciated that while two sets of studs **212** and holes **214** are shown, a single set, multiple sets or other arrangements could be used instead.

There are several advantages of the disclosed rigid and flexible strip electrodes over isolated and/or small footprint electrodes proposed in prior art. First, the surface area of each electrode is maximized to improve overall connectivity around the wrist. Second, since each electrode touches the skin all around the wrist, its reliability of coming in contact with the skin at all times (for example, during sleep) is significantly higher. Finally, by forming a connection all around the wrist, the dependence of each electrode's performance on its physical position around the wrist is minimized. Therefore, the smart band pair described in this disclosure, by virtue of its rigid and flexible strip electrodes, is able to acquire good quality ECG waveform data with a high degree of accuracy.

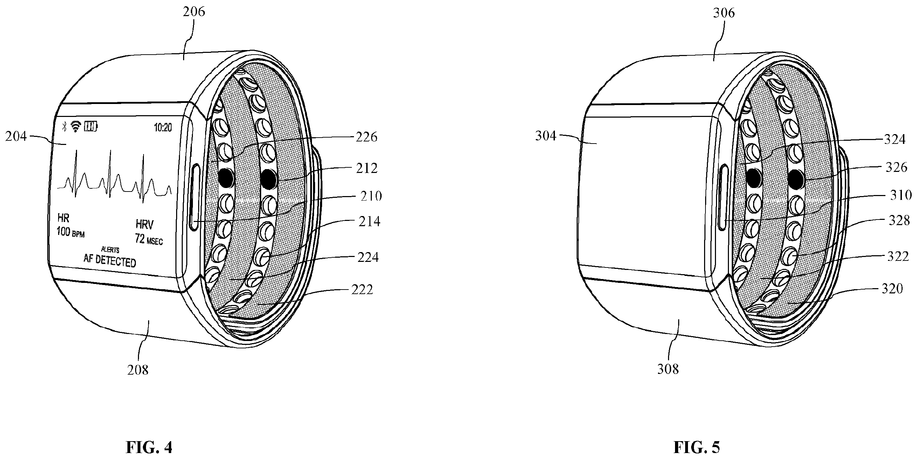

FIG. 4 illustrates an alternate view of the primary smart band showing the touchscreen display along with the straps, clasping mechanism, and flexible strip electrodes. The profile shape of the primary smart band straps **206, 208** is curved, and they provide a snug fit around the wrist using the stud **212** and hole **214** clasping mechanism. When worn around the wrist, the rigid strip electrodes (not shown) and the flexible strip electrodes **222, 224, 226** embedded in the straps **206, 208** make contact with the skin all around the wrist. The primary smart band is switched on by activating the on/off button **210.** The touchscreen display **204** helps in visualizing ECG waveform data and its analysis in real-time. In one example, the touchscreen display **204** displays real-time ECG waveform data along with HR/HRV metrics and pertinent alarms when these metrics are out of range. In another example, the user can interact with the touchscreen display **204** to perform tasks like reviewing historic ECG waveform data and/or sending a distress signal to other connected users/devices.

FIG. **5** illustrates an alternate view of the secondary smart band showing the front cover along with the straps, clasping mechanism, and flexible strip electrodes. The profile shape of the secondary smart band straps **306, 308** is curved, and they provide a snug fit around the wrist using the stud **326** and hole **328** clasping mechanism. When worn around the wrist, the rigid strip electrodes (not shown) and the flexible strip electrodes **320, 322, 324** embedded in the straps **306, 308** make contact with the skin all around the wrist. The secondary smart band is switched on by activating the on/off button **310.** In place of a touchscreen display, in this embodiment the secondary smart band is provided with a plastic front cover **304.**

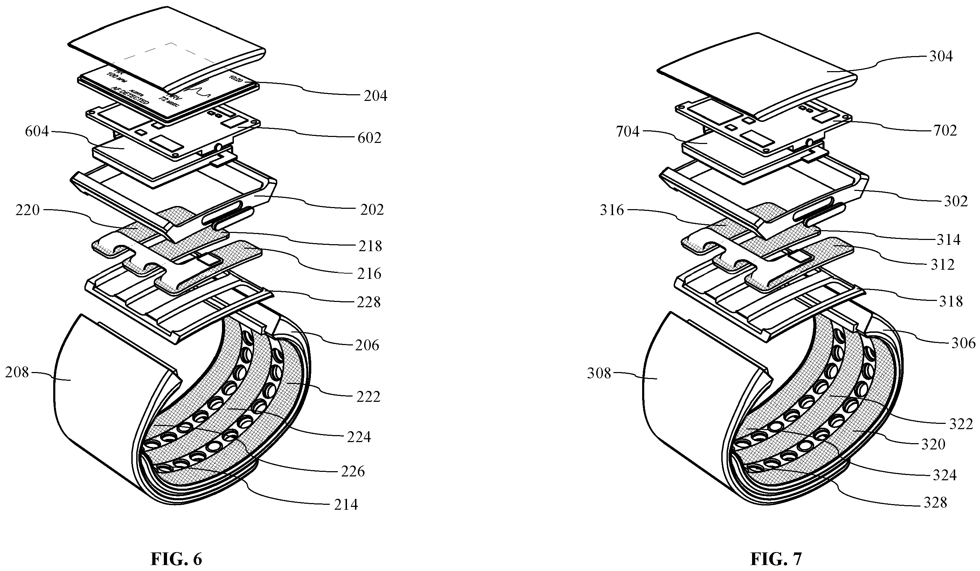

FIG. **6** illustrates an exploded view of the primary smart band showing the key components in one embodiment. These include a touchscreen display **204,** printed circuit board **602** containing all related hardware and running the desired software, enclosure **202,** rechargeable battery **604,** backplate **228** with embedded rigid strip electrodes **216, 218, 220** and straps **206, 208** with flexible strip electrodes **222, 224, 226** and clasping holes **214.**

FIG. **7** illustrates an exploded view of the secondary smart band showing the key components in one embodiment. These include a plastic front cover **304,** printed circuit board **702** containing all related hardware and running the desired software, enclosure **302,** rechargeable battery **704,** backplate **318** with embedded rigid strip electrodes **312, 314, 316** and straps **306, 308** with flexible strip electrodes **320, 322, 324** and clasping holes **328.**

In one example, desired components of the primary (FIG. 6) and secondary (FIG. 7) smart bands are provided with clipping mechanisms enabling them to be snap fitted.

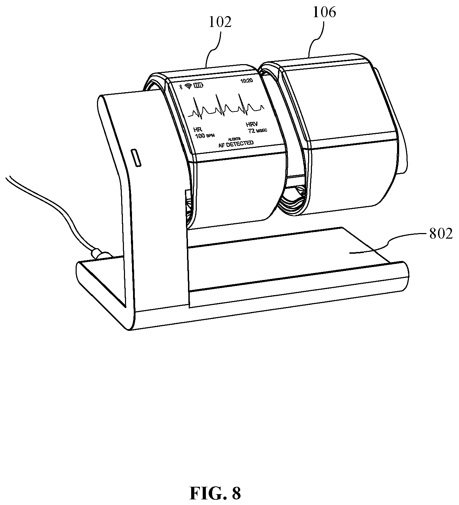

FIG. **8** illustrates the smart band pair being charged on a twin wireless charging unit. Both primary **102** and secondary **106** smart bands are provided with rechargeable batteries **604, 704** and wireless charging hardware/software. The smart band pair **102, 106** can therefore be charged on a twin wireless charging unit **802.** It will be appreciated that other charging arrangements, including wired, could also be used.

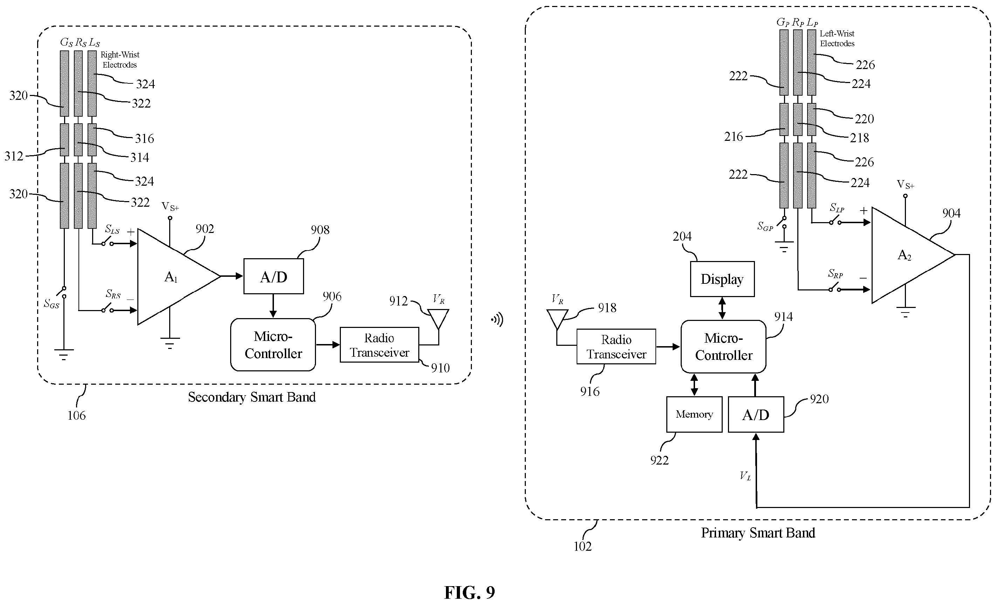

FIG. 9 illustrates an example operational diagram of the smart band pair whereby one electrode in each is a ground and biopotential data from both smart bands is sent directly to the microcontroller for processing. In this example, the secondary smart band **106** is attached to a user's right wrist and the primary smart band **102** is attached to the user's left wrist.

In FIG. 9, in the secondary smart band **106,** the three rigid strip electrodes **312, 314, 316** and the three flexible strip electrodes **320, 322, 324** are electrically connected. Similarly, in the primary smart band **102,** the three rigid strip electrodes **216, 218, 220** and the three flexible strip electrodes **222, 224, 226** are electrically connected.

Referring to FIG. 9, the secondary smart band **106** comprises three strip electrodes namely ground **312, 320,** right **314, 322,** and left **316, 324** electrodes that are connected to biopotential amplification and conditioning circuitry **902** via three digital switches $S_{GS}$, $S_{RS}$, $S_{LS}$. Similarly, the primary smart band **102** comprises three strip electrodes namely ground **216, 222,** right **218, 224,** and left **220, 226** electrodes that are connected to biopotential amplification and conditioning circuitry **904** via three digital switches $S_{GP}$, $S_{RP}$, $S_{LP}$.

As shown in FIG. 9, the right-side biopotential signal ($V_R$) measured by the secondary smart band ground **312, 320,** right **314, 322,** and left **316, 324** electrodes is acquired by the microcontroller **906** via an analog-to-digital (A/D) converter **908.** Using the radio transceiver **910** and antenna **912,** the microcontroller **906** wirelessly sends the right-side biopotential signal ($V_R$) to the primary smart band **102** attached to the user's left wrist. The primary smart band microcontroller **914** wirelessly receives the right-side biopotential signal ($V_R$) via its radio transceiver 916 and antenna **918.** At the same time, the left-side biopotential signal ($V_L$) measured by the primary smart band ground **216, 222,** right **218, 224,** and left **220, 226** electrodes is also acquired by the primary smart band microcontroller **914** via an A/D converter **920.** The primary smart band microcontroller **914** then processes and combines the biopotential signals $V_R$ and $V_L$ to produce a high-fidelity ECG signal. In its simplest form, a single-lead ECG signal ($V_I$) is synthesized by the smart band microcontroller **914** as per equation 1:

$$V_I = V_L - V_R \qquad\qquad (1)$$

In FIG. 9, the acquired ECG signal $V_I$ and related analytics are stored inside memory **922** and all related information is displayed in real-time on the touchscreen display **204** of the primary smart band **102.** Moreover, the primary smart band **102** also wirelessly transmits all ECG-related information via its radio transceiver **916** and antenna **918** to external devices **110** like smartphones, PCs, and tablets.

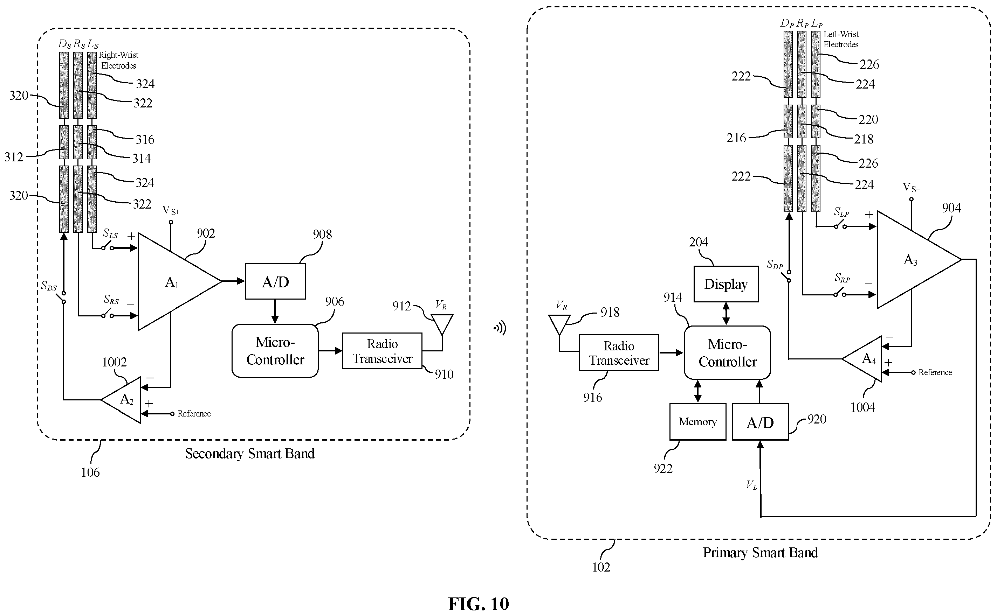

FIG. **10** illustrates an example operational diagram of the smart band pair whereby one electrode in each is an RLD and biopotential data from both smart bands is sent directly to the microcontroller for processing. In this example, the secondary smart band **106** is attached to user's right wrist and the primary smart band **102** is attached to user's left wrist.

In FIG. **10,** the secondary smart band strip electrodes **312, 320, 314, 322, 316, 324** and the primary smart band strip electrodes **216, 222, 218, 224, 220, 226** are connected in a manner similar to that described for FIG **9.**

As shown in FIG. **10,** the secondary smart band **106** comprises three strip electrodes namely RLD **312, 320,** right **314, 322,** and left **316, 324** electrodes that are connected to biopotential amplification/conditioning circuitry **902** and RLD circuitry **1002** via three digital switches $S_{DS}$, $S_{RS}$, $S_{LS}$. Similarly, the primary smart band **102** comprises three strip electrodes namely RLD **216, 222,** right **218, 224,** and left **220, 226** electrodes that are connected to biopotential amplification/conditioning circuitry **904** and RLD circuitry **1004** via three digital switches $S_{DP}$, $S_{RP}$, $S_{LP}$.

Referring to FIG. **10,** the method for single-lead ECG waveform data acquisition is similar to that described for FIG. **9.** The only difference is that the quality and fidelity of the acquired ECG signal is further augmented by virtue of the RLD electrodes **312, 320, 216, 222** and circuitry **1002, 1004** that minimize common mode noise.

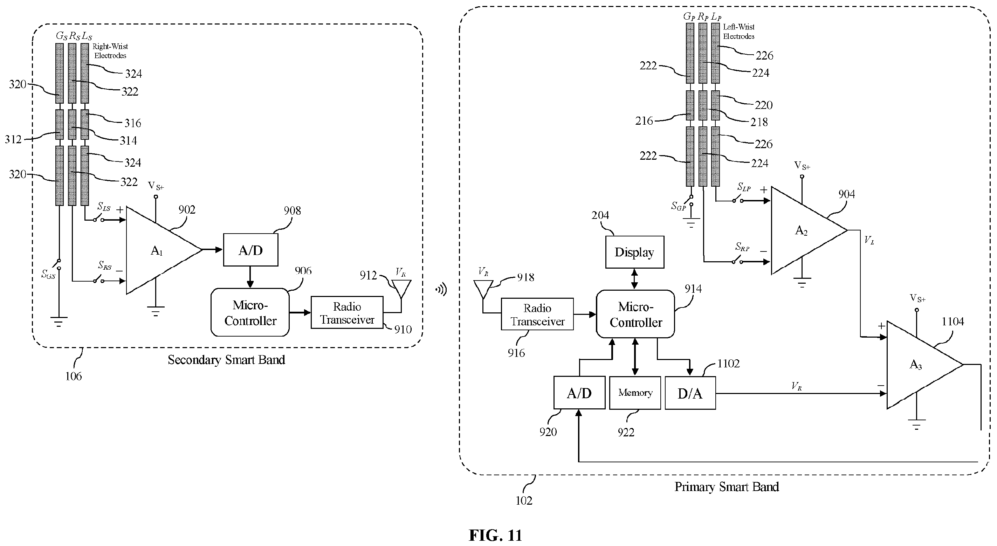

FIG. **11** illustrates an example operational diagram of the smart band pair whereby one electrode in each is a ground and biopotential data from both smart bands is first sent to a differential amplifier and then to the microcontroller for processing. In this example, the secondary smart band **106** is attached to user's right wrist and the primary smart band **102** is attached to user's left wrist.

In FIG. 11, the secondary smart band strip electrodes **312, 320, 314, 322, 316, 324** and the primary smart band strip electrodes **216, 222, 218, 224, 220, 226** are connected in a manner similar to that described for FIG **9.**

As shown in FIG. **11,** the secondary smart band **106** comprises three strip electrodes namely ground **312, 320,** right **314, 322,** and left **316, 324** electrodes that are connected to biopotential amplification and conditioning circuitry **902** via three digital switches $S_{GS}$, $S_{RS}$, $S_{LS}$. Similarly, the primary smart band **102** comprises three strip electrodes namely ground **216, 222,** right **218, 224,** and left **220, 226** electrodes that are connected to biopotential amplification and conditioning circuitry **904** via three digital switches $S_{GP}$, $S_{RP}$, $S_{LP}$.

Referring to FIG. 11, similar to the method described for FIG. 9, the primary smart band microcontroller **914** wirelessly receives the right-side biopotential signal ($V_R$) via its radio transceiver **916** and antenna **918.** The microcontroller **914** then sends this signal via a digital-to-analog (D/A) converter **1102** to a differential amplifier **1104.** At the same time, this differential amplifier **1104** also receives the left-side biopotential signal ($V_L$) measured by the primary smart band strip electrodes **216, 222, 218, 224, 220, 226** in conjunction with the biopotential amplification and conditioning circuitry **904.** The primary smart band microcontroller **914** then acquires the amplified ECG signal output from the differential amplifier **1104** via the A/D converter **920.** This highlights another method for amplifying the biopotential signals $V_R$ and $V_L$ for obtaining a high-quality ECG signal.

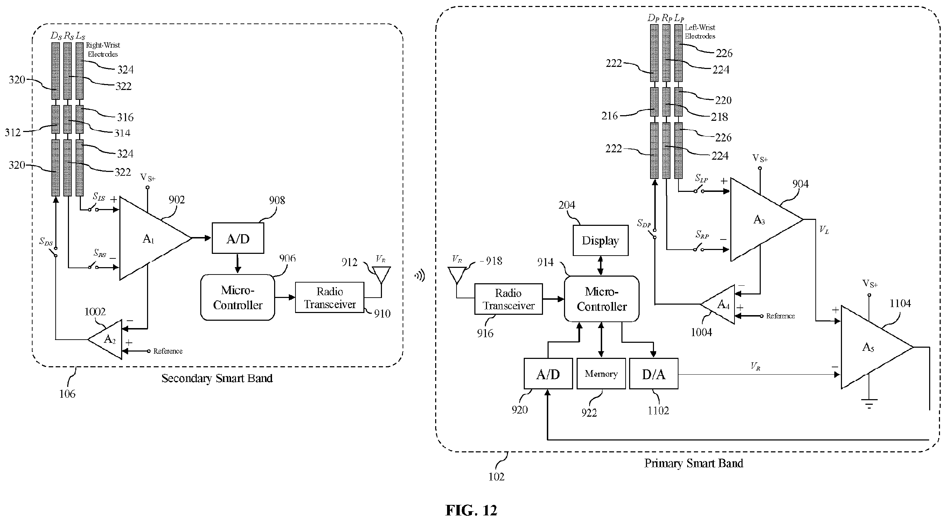

FIG. **12** illustrates an example operational diagram of the smart band pair whereby one electrode in each is an RLD and biopotential data from both smart bands is first sent to a differential amplifier and then to the microcontroller for processing. In this example, the secondary smart band **106** is attached to user's right wrist and the primary smart band **102** is attached to user's left wrist.

In FIG. **12,** the secondary smart band strip electrodes **312, 320, 314, 322, 316, 324** and the primary smart band strip electrodes **216, 222, 218, 224, 220, 226** are connected in a manner similar to that described for FIG 9.

As shown in FIG. **12,** the secondary smart band **106** comprises three strip electrodes namely RLD **312, 320,** right **314, 322,** and left **316, 324** electrodes that are connected to biopotential amplification/conditioning circuitry **902** and RLD circuitry **1002** via three digital switches $S_{DS}$, $S_{RS}$, $S_{LS}$. Similarly, the primary smart band **102** comprises three strip electrodes namely RLD **216, 222,** right **218, 224,** and left **220, 226** electrodes that are connected to biopotential amplification/conditioning circuitry **904** and RLD circuitry **1004** via three digital switches $S_{DP}$, $S_{RP}$, $S_{LP}$.

Referring to FIG. **12,** the method for single-lead ECG waveform data acquisition is similar to that described for FIG. **11.** The only difference is that the quality and fidelity of the acquired ECG signal is further augmented by virtue of the RLD electrodes **312, 320, 216, 222** and circuitry **1002, 1004** that minimize common mode noise.

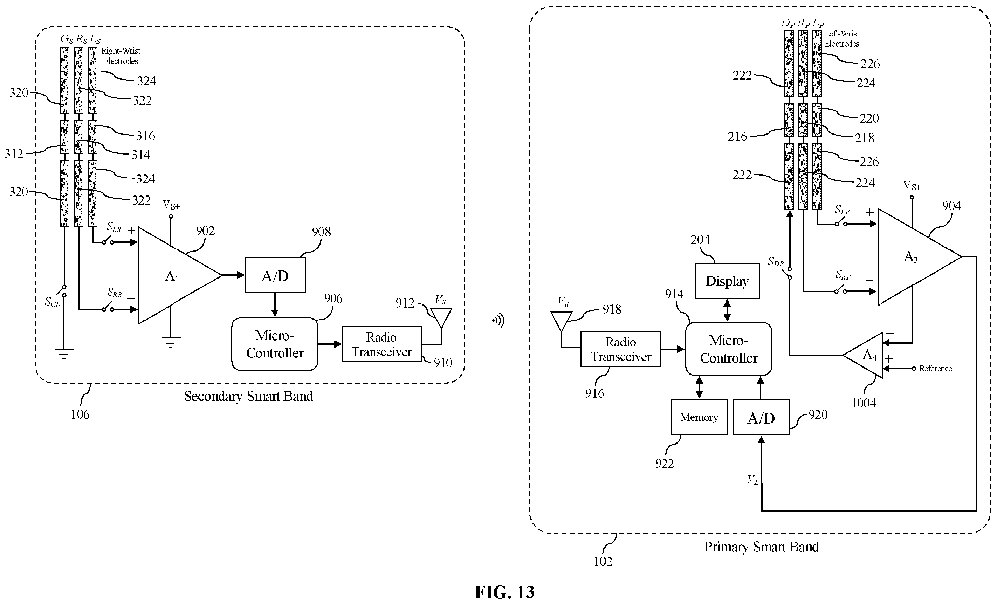

FIG. **13** illustrates an example operational diagram of the smart band pair whereby one electrode in the secondary is a ground while one electrode in the primary is an RLD and biopotential data from both smart bands is sent directly to the microcontroller for processing. In this example, the secondary smart band **106** is attached to user's right wrist and the primary smart band **102** is attached to user's left wrist.

In FIG. **13,** the secondary smart band strip electrodes **312, 320, 314, 322, 316, 324** and the primary smart band strip electrodes **216, 222, 218, 224, 220, 226** are connected in a manner similar to that described for FIG **9.**

As shown in FIG. **13,** the secondary smart band **106** comprises three strip electrodes namely ground **312, 320,** right **314, 322,** and left **316, 324** electrodes that are connected to biopotential amplification/conditioning circuitry **902** via three digital switches $S_{GS}$, $S_{RS}$, $S_{LS}$. On the other hand, the primary smart band **102** comprises three strip electrodes namely RLD **216, 222,** right **218, 224,** and left **220, 226** electrodes that are connected to biopotential amplification/conditioning circuitry **904** and RLD circuitry **1004** via three digital switches $S_{DP}$, $S_{RP}$, $S_{LP}$.

Referring to FIG. **13,** the method for single-lead ECG waveform data acquisition is similar to that described for FIG. **9.** The only difference is that the secondary smart band **106** comprises a ground electrode **312, 320** while the primary smart band **102** comprises an RLD electrode **216, 222** to further reduce noise and enhance quality and fidelity of the acquired ECG signal.

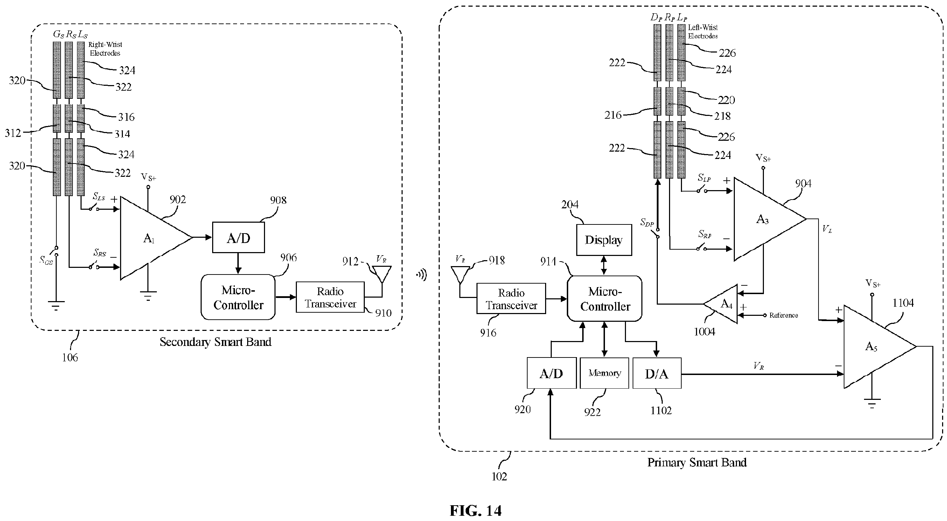

FIG. **14** illustrates an example operational diagram of the smart band pair whereby one electrode in the secondary is a ground while one electrode in the primary is an RLD and biopotential data from both smart bands is first sent to a differential amplifier and then to the microcontroller for processing. In this example, the secondary smart band **106** is attached to user's right wrist and the primary smart band **102** is attached to user's left wrist.

In FIG. **14,** the secondary smart band strip electrodes **312, 320, 314, 322, 316, 324** and the primary smart band strip electrodes **216, 222, 218, 224, 220, 226** are connected in a manner similar to that described for FIG **9.**

As shown in FIG. **14,** the secondary smart band **106** comprises three strip electrodes namely ground **312, 320,** right **314, 322,** and left **316, 324** electrodes that are connected to biopotential amplification/conditioning circuitry **902** via three digital switches $S_{GS}$, $S_{RS}$, $S_{LS}$. On the other hand, the primary smart band **102** comprises three strip electrodes namely RLD **216, 222,** right **218, 224,** and left **220, 226** electrodes that are connected to biopotential amplification/conditioning circuitry 904 and RLD circuitry **1004** via three digital switches $S_{DP}$, $S_{RP}$, $S_{LP}$.

Referring to FIG. **14,** the method for single-lead ECG waveform data acquisition is similar to that described for FIG. **11.** The only difference is that the secondary smart band **106** comprises a ground electrode **312, 320** while the primary smart band **102** comprises an RLD electrode **216, 222** to further reduce noise and enhance quality and fidelity of the acquired ECG signal.

In the examples shown in FIGS. **9-14,** switches $S_{GP}/S_{DP}$, $S_{RP}$, $S_{LP}$ are provided for primary smart band strip electrodes **216, 222, 218, 224, 220, 226** and switches $S_{GS}/S_{DS}$, $S_{RS}$, $S_{LS}$ are provided for secondary smart band strip electrodes **312, 320, 314, 322, 316, 324.** This allows for different electrode configurations and connections to be used for each smart band to minimize signal-to-noise ratio (SNR), thus further enhancing ECG signal quality. This feature is useful for device testing and calibration whereby related hardware/software can be fine-tuned to obtain optimum signal quality.

A truth table in Table 1 below summarizes all possible electrode configurations and connections that can be used on each smart band to optimize and augment ECG signal quality. In Table 1, $S_G$ is the ground electrode switch, $S_D$ is the RLD electrode switch, $S_L$ is the left electrode switch, and $S_R$ is the right electrode switch of the primary and/or secondary smart band. The on state of an electrode switch is represented by true (*T*) while the off state of an electrode switch is represented by false (*F*).

| $S_G/S_D$ | $S_L$ | $S_R$ |
|---|---|---|
| T | F | F |
| F | T | F |
| F | F | T |
| T | T | F |

(continued)

| $S_G/S_D$ | $S_L$ | $S_R$ |
|-----------|-------|-------|
| T | F | T |
| F | T | T |
| T | T | T |
| F | F | F |

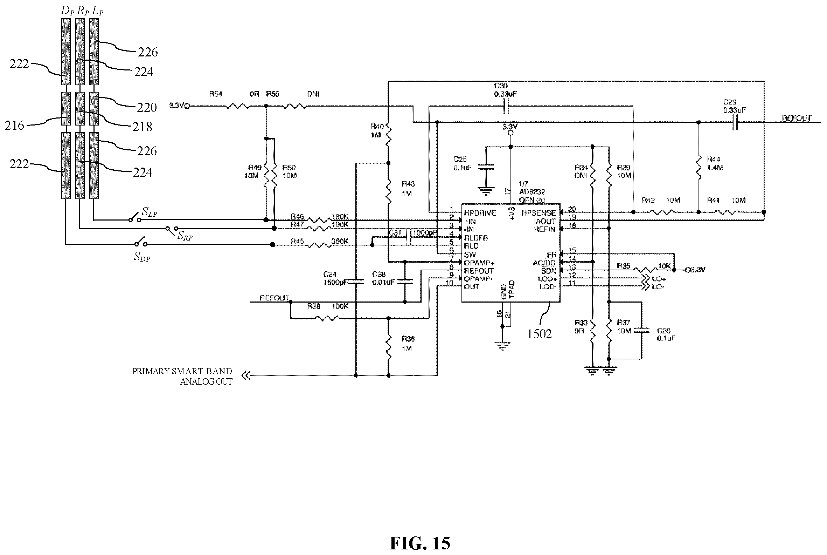

FIG. **15** illustrates an example circuit diagram of a biopotential amplifier with an RLD strip electrode implemented using Analog Devices AD8232 chip. The biopotential amplifiers described in FIG. 9-**14,** can be easily implemented using commercially available ECG analog front ends like the AD8232 chip **1502**. The disclosed circuit diagram shows the values of various electronic components and the primary smart band strip electrodes **216, 222, 218, 224, 220, 226** connected to the AD8232 chip **1502**. In this case, the primary smart band strip electrodes **216, 222** are RLD electrodes since they are connected to an RLD circuitry inside the AD8232 chip **1502**.

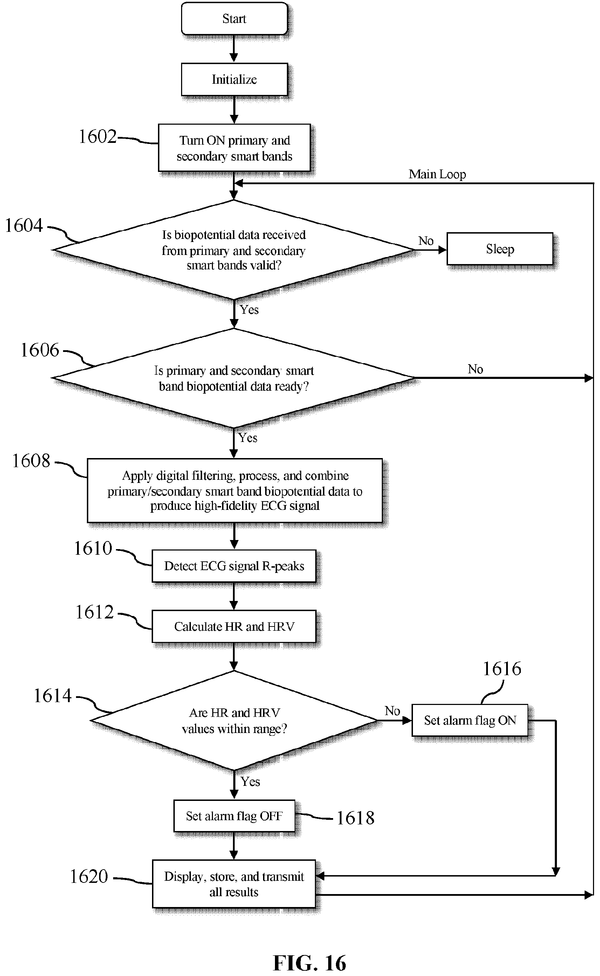

FIG. **16** illustrates a flowchart depicting one example method of continuous ECG monitoring and HR/HRV analysis whereby biopotential data from both smart bands is sent directly to the microcontroller for processing. At step **1602** both primary **102** and secondary **106** smart bands are switched on using buttons **210** and **310**. At step **1604** the microcontroller **914** inside the primary smart band checks whether biopotential data that is wirelessly received from the secondary smart band **106** and the biopotential data that is received via A/D converter of the primary smart band **102** is valid. If the data in step **1604** is found to be valid, the primary smart band **102** waits for this data to be ready for processing at step **1606.** Once all biopotential data is ready, the primary smart band microcontroller **914** performs various analyses like digital filtering and other mathematical operations on this data at step **1608** to produce high-fidelity ECG waveform data. At step **1610,** the primary smart band microcontroller **914** detects ECG R-peaks and then at step **1612** it computes metrics like HR and HRV. In this example, at step **1614,** the primary smart band microcontroller **914** checks the calculated HR/HRV metrics against predefined acceptable values. Based on whether the calculated HR/HRV parameters are in range or out of range, alarm flags are accordingly set at steps **1616** and **1618**. At step **1620,** the primary smart band touchscreen display **204** shows ECG waveform data and related analytics along with the alarm status in real-time. Moreover, at step **1620,** the primary smart band wirelessly transmits all ECG waveform data and related analytics to third-party devices **110**.

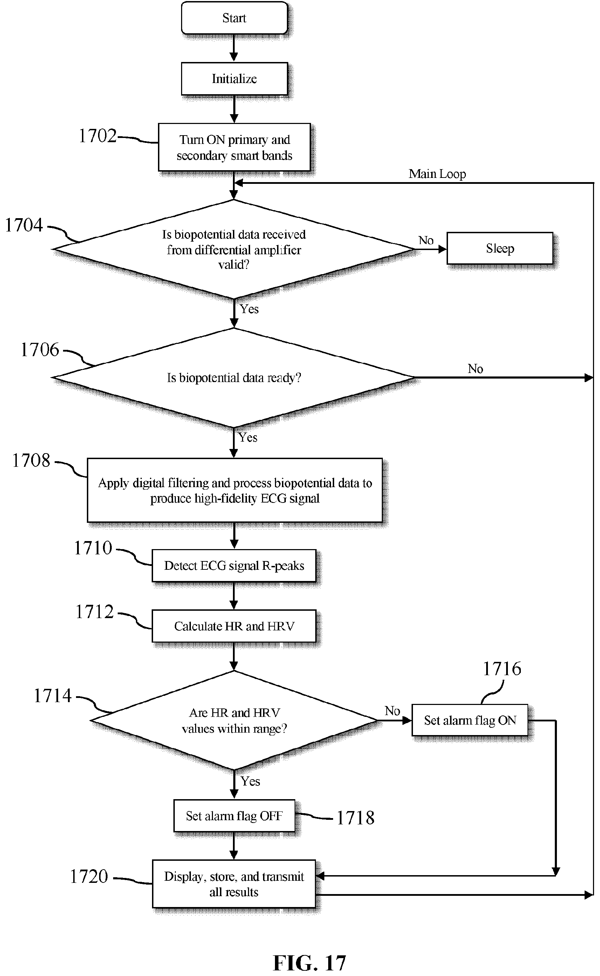

FIG. **17** illustrates an example flowchart depicting the method of continuous ECG monitoring and HR/HRV analysis whereby biopotential data from both smart bands is first sent to a differential amplifier and then to the microcontroller for processing. This flowchart describes a method for ECG waveform data acquisition and analysis that is similar to that described for FIG. **16.** The main difference is that at step **1704** the primary smart band microcontroller **914** checks for the validity of the amplified primary and secondary smart band biopotential data that it receives from the differential amplifier **1104** inside the primary smart band **102**.

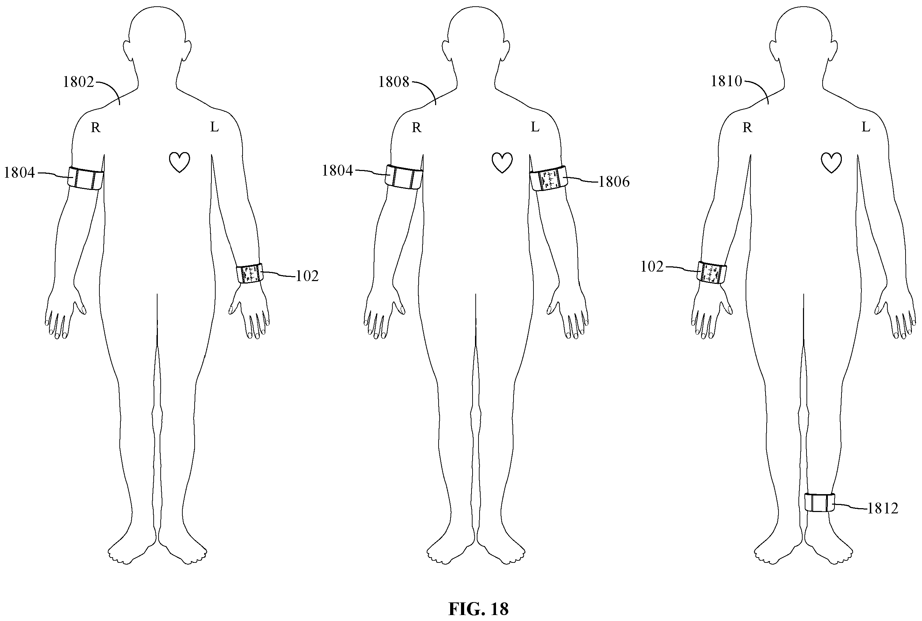

FIG. **18** illustrates examples of various locations on the human body where wearables employing the underlying design and principle of the current invention can be attached to undertake continuous leadless ECG monitoring. As illustrated at **1802,** the primary smart band **102** can be worn around the left wrist while a secondary smart band **1804** can be worn around the right upper arm. As illustrated at **1808,** a primary smart band **1806** can be worn around the left upper arm while the secondary smart band **1804** can be worn around the right upper arm. Finally, as illustrated at **1810,** the primary smart band **102** can be worn around the right wrist while a secondary smart band **1812** can be worn around the left ankle. These examples demonstrate that the disclosed wireless smart band pair and/or other similar wearable pair can be attached at various locations along the four limbs to accomplish leadless Einthoven-type single-lead ECG measurements.

Fusion of DSP and Analog Signal Conditioning Techniques

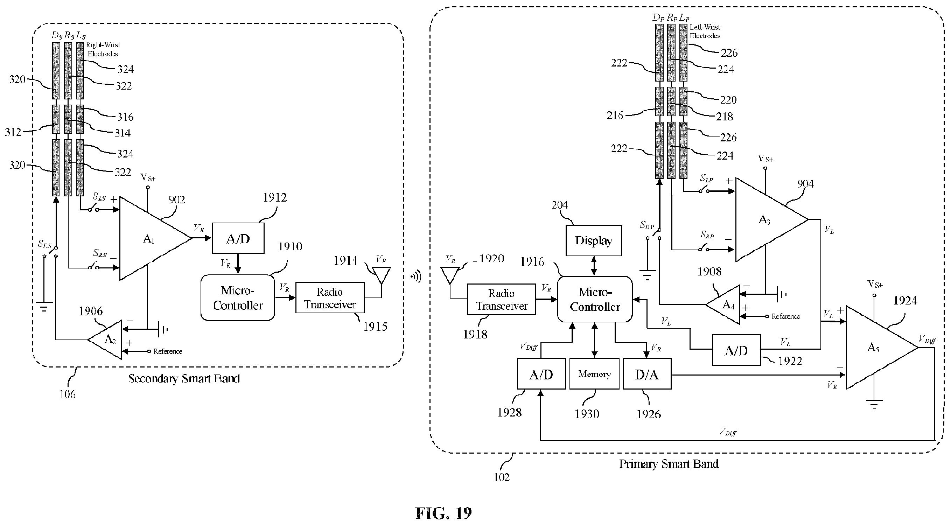

FIG. **19** illustrates an example operational diagram of the smart band pair. Here, the reference electrode in each smart band is either a ground or RLD. Biopotential data from both smart bands is sent directly and also via a differential amplifier to the primary smart band's microcontroller for processing. In this example, the secondary smart band **106** is attached to a user's right wrist while the primary smart band **102** is attached to the user's left wrist.

Referring to FIG. 19, the secondary smart band **106** comprises three strip electrodes namely ground or RLD **312, 320,** right **314, 322,** and left **316, 324** electrodes that are connected to biopotential amplification and conditioning circuitry **902** via three digital switches $S_{DS}$, $S_{RS}$, $S_{LS}$. Similarly, the primary smart band **102** comprises three strip electrodes namely ground or RLD **216, 222,** right **218, 224,** and left **220, 226** electrodes that are connected to biopotential amplification and conditioning circuitry **904** via three digital switches $S_{DP}$, $S_{RP}$, $S_{LP}$. The secondary smart band switches $S_{DS}$, $S_{RS}$, $S_{LS}$ are controlled by the secondary smart band microcontroller **1910** while the primary smart band switches $S_{DP}$, $S_{RP}$, $S_{LP}$ are controlled by the primary smart band microcontroller **1916**.

In FIG. 19, $S_{DP}$ in the primary smart band **102** and $S_{DS}$ in the secondary smart band **106** are changeover switches with two binary states, namely, 0 and 1. In state 0, they convert their respective electrodes to ground electrodes via grounding whereas in state 1, they convert their respective electrodes to RLD electrodes via the amplifiers **1906** and **1908.** This allows for various combinations of ground and RLD electrodes to be readily used in the primary and secondary smart bands to reduce noise and enhance ECG signal quality.

Referring to FIG. 19, switches $S_{RP}$ and $S_{LP}$ are provided for primary smart band strip electrodes **218, 224, 220, 226** and switches $S_{RS}$ and $S_{LS}$ are provided for secondary smart band strip electrodes **314, 322, 316, 324.** Again, these switches have two binary states, namely, 0 and 1. A state 0 will remove these electrodes from the ECG monitoring circuit whereas a state 1 will connect these electrodes to the ECG monitoring circuit. This allows for different electrode configurations and connections to be used for each smart band to minimize signal-to-noise ratio (SNR), thus further enhancing ECG signal quality.

In one example, if states of $S_{DS}$, $S_{LS}$, $S_{RS}$, $S_{DP}$, $S_{LP}$, and $S_{RP}$ are 1, then secondary and primary smart band strip electrodes **314, 322, 316, 324, 218, 224, 220,** and **226** will be involved in ECG waveform data monitoring wherein the reference electrodes **312, 320, 216,** and **222** will act as RLD electrodes. In another example, if states of $S_{DS}$, $S_{LS}$, and $S_{RP}$ are 0 while states of $S_{RS}$, $S_{DP}$, and $S_{LP}$ are 1, then secondary and primary smart band strip electrodes **314, 322, 220,** and **226** will be involved in ECG waveform data monitoring wherein the reference electrodes **312, 320** will act as ground electrodes and reference electrodes **216, 222** will act as RLD electrodes. In addition to reducing noise, the switching feature is also very useful for device testing and calibration whereby related hardware/software can be fine-tuned to obtain optimum signal quality.

As shown in FIG. 19, the right-side biopotential signal ($V_R$) measured by the secondary smart band electrodes **312, 320, 314, 322, 316,** and **324** is acquired by the microcontroller **1910** via an analog-to-digital (A/D) converter **1912.** Using the radio transceiver **1915** and antenna **1914,** the microcontroller **1910** wirelessly sends the right-side biopotential signal ($V_R$) to the primary smart band **102** attached to the user's left wrist. The primary smart band microcontroller **1916** wirelessly receives the right-side biopotential signal ($V_R$) via its radio transceiver **1918** and antenna **1920.** At the same time, the left-side biopotential signal ($V_L$) measured by the primary smart band electrodes **216, 222, 218, 224, 220, 226** is also acquired by the primary smart band microcontroller **1916** via an A/D converter **1922.** Additionally, the left-side biopotential signal ($V_L$) is fed to the first terminal of a differential amplifier **1924** inside the primary smart band **102.** Moreover, the right-side biopotential signal ($V_R$) from the primary smart band microcontroller **1916** is fed via a D/A converter **1926** to the second terminal of the differential amplifier **1924.** The differential amplifier **1924** output ($V_{Diff}$) is then acquired by the primary smart band microcontroller **1916** via the A/D converter **1928.**

In reference with FIG. **19,** the primary smart band microcontroller **1916** employs various DSP techniques on the biopotential signals $V_R$ and $V_L$ to produce a high-fidelity ECG signal. Based on equation 1, in one example, a single-lead ECG signal ($ECG_{Digital}$) is synthesized by the primary smart band microcontroller **1916** by computing the difference between the biopotential signals $V_R$ and $V_L$ as per equation 2:

$$ECG_{Digital} = (V_L - V_R) \qquad (2)$$

In another example, a single-lead ECG signal ($ECG_{Digital}$) is synthesized by the primary smart band microcontroller **1916** by computing the weighted mean of the biopotential signals $V_R$ and $V_L$ using respective weights $W_R$ and $W_L$ as per equation 3:

$$ECG_{Digital} = \frac{(W_L V_L + W_R V_R)}{(W_L + W_R)} \qquad (3)$$

In yet another example, a single-lead ECG signal ($ECG_{Digital}$) is synthesized by the primary smart band microcontroller 1916 by computing a convolution between the biopotential signals $V_R$ and $V_L$ as per equation 4, whereby n is the number of samples in the $V_R$ and $V_L$ arrays:

$$ECG_{Digital}[n] = V_R[n] * V_L[n] = \sum_{k=-\infty}^{\infty} V_R[k].V_L[n-k] \quad (4)$$

As per FIG. 19, the primary smart band microcontroller **1916** also receives the signal $V_{Diff}$, which is the result of the analog signal amplification and conditioning of $V_R$ and $V_L$ via the differential amplifier **1924.** Therefore, the high-fidelity analog ECG signal ($ECG_{Analog}$), can be defined via equation 5 as follows:

$$ECG_{Analog} = V_{Diff} \qquad (5)$$

$ECG_{Digital}$ (equations (2)-(4) and FIG. 19) and $ECG_{Analog}$ (equation (5) and FIG. 19) represent high-fidelity ECG signals that are obtained via two very distinct and complementary techniques - DSP and analog signal conditioning respectively.

In one example, the primary smart band microcontroller **1916** (FIG. 19) combines and fuses the $ECG_{Digital}$ and $ECG_{Analog}$ signals to further suppress noise and obtain an even higher quality and fidelity signal, namely, $ECG_{Fusion}$.

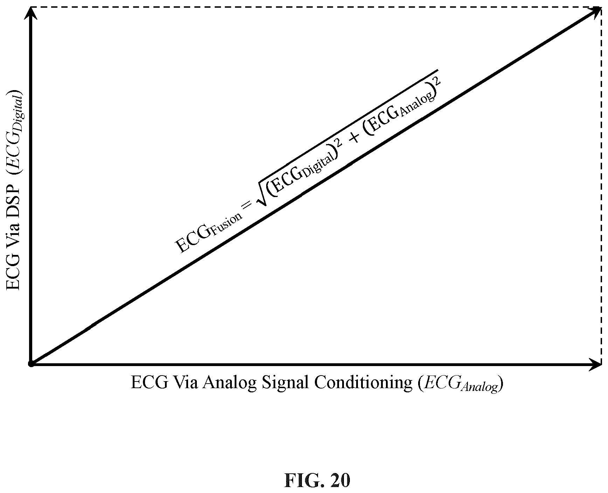

The concept of fusion of complementary ECG signals ($ECG_{Digital}$ and $ECG_{Analog}$) to obtain a higher fidelity ECG signal ($ECG_{Fusion}$) can be explained via FIG. **20** and represented by equation 6:

$$ECG_{Fusion} = \sqrt{\left(ECG_{Digital}\right)^2 + \left(ECG_{Analog}\right)^2} \qquad (6)$$

There are several other ways by which $ECG_{Fusion}$ can be computed. In one example, $ECG_{Fusion}$ is computed as an arithmetic mean of $ECG_{Digital}$ and $ECG_{Analog}$ as per equation (7):

$$ECG_{Fusion} = \frac{ECG_{Digital} + ECG_{Analog}}{2} \qquad (7)$$

The biopotential amplifiers described in FIG. 19, can be easily implemented using commercially available ECG analog front ends like the AD8232 chip **1102.** The disclosed circuit diagram of FIG. 15 shows the values of various electronic components and the primary smart band strip electrodes **216, 222, 218, 224, 220, 226** connected to the AD8232 chip **1102.**

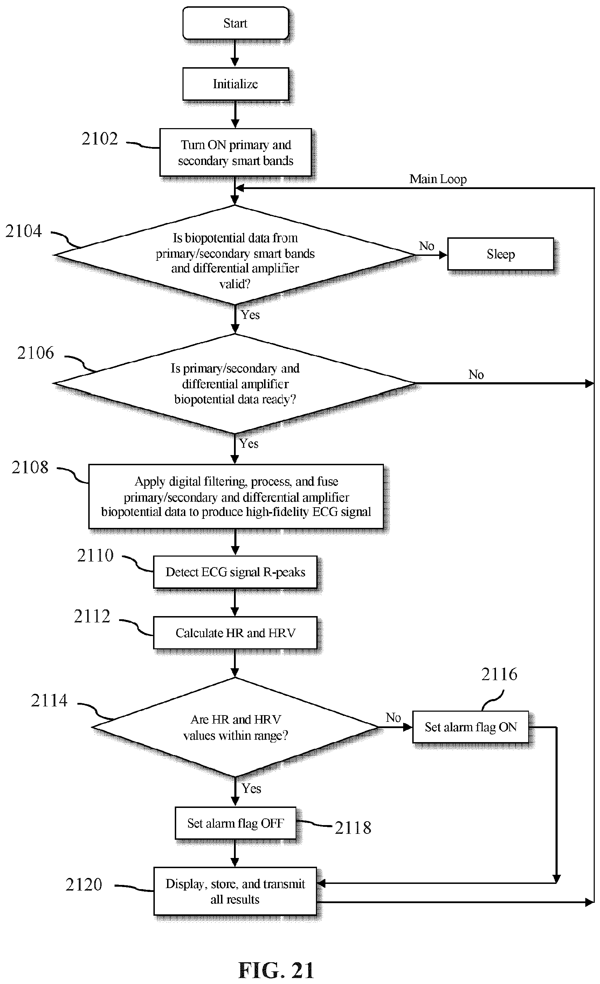

FIG. **21** illustrates a flowchart depicting one example method of continuous high-fidelity ECG monitoring and HR/HRV analysis via fusion of two complementary techniques, namely, DSP and analog signal conditioning. At step **2102** both primary **102** and secondary **106** smart bands are switched on using buttons **210** and **310.** At step **2104** the microcontroller **1916** inside the primary smart band checks whether biopotential data $V_R$ that is wirelessly received from the secondary smart band **106,** the biopotential data $V_L$ that is received from amplifier **904** via A/D converter **1922,** and biopotential data $V_{Diff}$ that is received from the differential amplifier **1924** via A/D converter **1928** is valid. If all data in step **2104** is found to be valid, the primary smart band **102** waits for this data to be ready for processing at step **2106.** Once all biopotential data is ready, the primary smart band microcontroller **1916** performs various computations like digital filtering, differencing, convolution, fusion, and other mathematical operations on this data at step **2108** to produce high-fidelity ECG waveform data. At step **2110,** the primary smart band microcontroller **1916** detects ECG R-peaks and then at step **2112** it computes metrics like HR and HRV. In this example, at step **2114,** the primary smart band microcontroller **1916** checks the calculated HR/HRV metrics against predefined acceptable values. Based on whether the calculated HR/HRV parameters are in range or out of range, alarm flags are accordingly set at steps **2116** and **2118.** At step **2120,** the primary smart band touchscreen **204** displays ECG waveform data and related analytics along with the alarm status in real-time. Moreover, at step **2120,** the primary smart band wirelessly transmits all ECG waveform data and related analytics to third-party devices **110.**

<u>Electrode Switching</u>

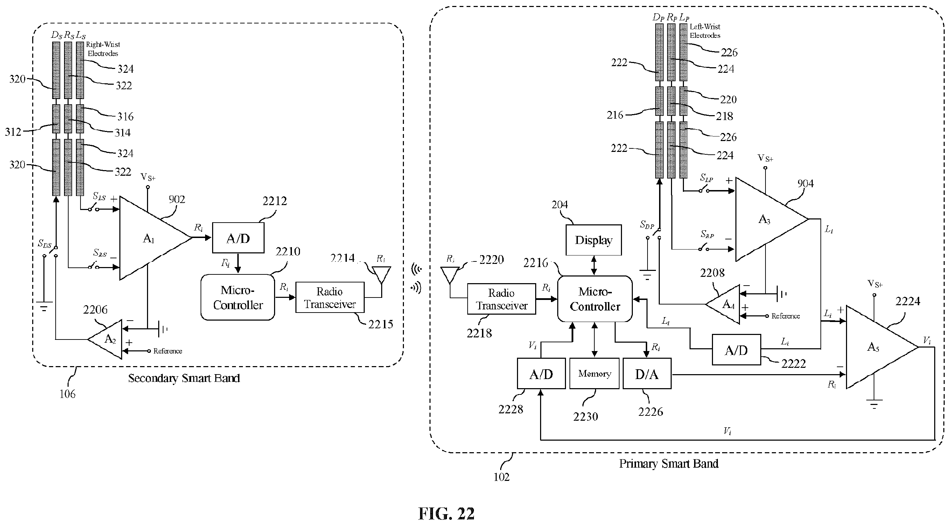

FIG. **22** illustrates an example operational diagram of the smart band pair. Here, the reference electrode in each smart band is either a ground or RLD. Biopotential data from both smart bands is sent directly and also via a differential amplifier to the primary smart band's microcontroller for processing. In this example, the secondary smart band **106** is attached to a user's right wrist while the primary smart band **102** is attached to the user's left wrist.

In FIG. **22,** in the secondary smart band **106,** the three rigid strip electrodes **312, 314, 316** and the three flexible strip electrodes **320, 322, 324** are electrically connected. Similarly, in the primary smart band **102,** the three rigid strip electrodes **216, 218, 220** and the three flexible strip electrodes **222, 224, 226** are electrically connected.

Referring to FIG. **22,** the secondary smart band **106** comprises three strip electrodes namely ground or RLD **312, 320,** right **314, 322,** and left **316, 324** electrodes that are connected to biopotential amplification and conditioning circuitry **902** via three digital switches $S_{DS}$, $S_{RS}$, $S_{LS}$. Similarly, the primary smart band **102** comprises three strip electrodes namely ground or RLD **216, 222,** right **218, 224,** and left **220, 226** electrodes that are connected to biopotential amplification and conditioning circuitry **904** via three digital switches $S_{DP}$, $S_{RP}$, $S_{LP}$. The secondary smart band switches $S_{DS}$, $S_{RS}$, $S_{LS}$ are controlled by the secondary smart band microcontroller **2210** while the primary smart band switches $S_{DP}$, $S_{RP}$, $S_{LP}$ are controlled by the primary smart band microcontroller **2216.**

In FIG. **22,** $S_{DP}$ in the primary smart band **102** and $S_{DS}$ in the secondary smart band **106** are changeover switches with two binary states, namely, 0 and 1. In state 0, they convert their respective electrodes to ground electrodes via grounding whereas in state 1, they convert their respective electrodes to RLD electrodes via the amplifiers **2206** and **2208.** This feature allows for various combinations of ground and RLD electrodes to be readily used in the primary and secondary smart bands to reduce noise and enhance ECG signal quality.

Referring to FIG. **22,** switches $S_{RP}$ and $S_{LP}$ are provided for primary smart band strip electrodes **218, 224, 220, 226** and switches $S_{RS}$ and $S_{LS}$ are provided for secondary smart band strip electrodes **314, 322, 316, 324.** Again, these switches have two binary states, namely, 0 and 1. A state 0 will remove these electrodes from the ECG monitoring circuit whereas a state 1 will connect these electrodes to the ECG monitoring circuit. This allows for different electrode configurations and connections to be used for each smart band to minimize signal-to-noise ratio (SNR), thus further enhancing ECG signal quality.

In one example, if states of $S_{DS}$, $S_{LS}$, $S_{RS}$, $S_{DP}$, $S_{LP}$, and $S_{RP}$ are 1, then secondary and primary smart band strip electrodes **314, 322, 316, 324, 218, 224, 220,** and **226** will be involved in ECG waveform data monitoring wherein the reference electrodes **312, 320, 216,** and **222** will act as RLD electrodes. In another example, if states of $S_{DS}$, $S_{LS}$, and $S_{RP}$ are 0 while states of $S_{RS}$, $S_{DP}$, and $S_{LP}$ are 1, then secondary and primary smart band strip electrodes **314, 322, 220,** and **226** will be involved in ECG waveform data monitoring wherein the reference electrodes **312, 320** will act as ground electrodes and reference electrodes **216, 222** will act as RLD electrodes. In addition to reducing noise, the switching feature is also very useful for device testing and calibration whereby related hardware/software can be fine-tuned to obtain optimum signal quality.

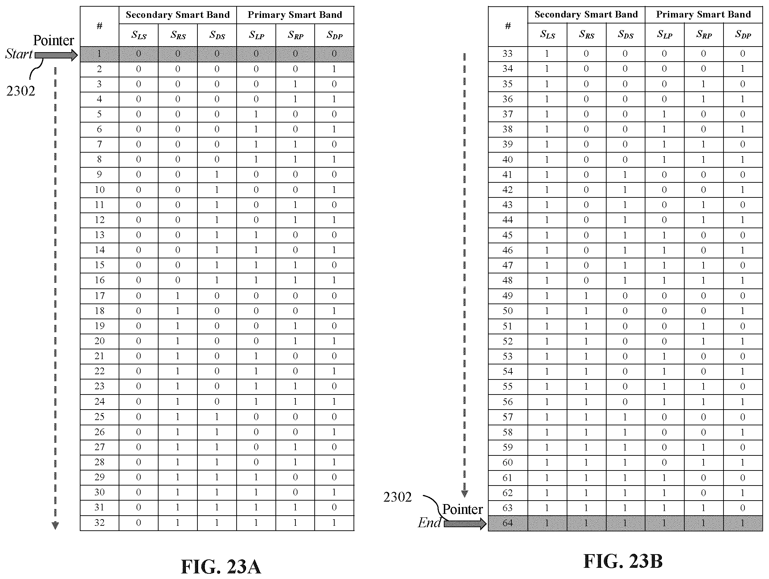

In one embodiment, rapid switching of all six switches, namely, $S_{DS}$, $S_{LS}$, $S_{RS}$, $S_{DP}$, $S_{LP}$, and $S_{RP}$ is undertaken in such a manner that left and right side biopotential data is acquired, processed, and combined every data sampling period as the system continuously and repeatedly loops through all possible configurations of the switches. Since each switch has 2 states (0 and 1) and total number of switches are 6, there are $2^6 = 64$ unique configurations that are possible per data sampling period (FIGS. **23A-23B**).

In one example, a truth table of the type shown in FIGS. **23A-23B** is stored inside the primary smart band memory **2230** with the pointer **2302** at configuration # 1. As per configuration # 1 row, the desired states of the secondary smart band switches $S_{LS}$, $S_{RS}$, and $S_{DS}$ are wirelessly transmitted by the primary smart band microcontroller **2216** to the secondary smart band microcontroller **2210** employing radio transceivers **2218, 2212** and antennas **2220, 2214.** The secondary smart band microcontroller **2210** then sets the states of switches $S_{LS}$, $S_{RS}$, and $S_{DS}$ based on the information received from the primary smart band microcontroller **2216.** At the same time, the primary smart band microcontroller **2216** sets the states of switches $S_{LP}$, $S_{RP}$, and $S_{DP}$ as per configuration # 1 row. Once all six switches are set to the states described by configuration # 1 row, the secondary smart band **106** acquires biopotential data from the right wrist and transmits it wirelessly to the primary smart band **102** that also simultaneously acquires biopotential data from the from the left wrist. Then, the primary smart band microcontroller **2216** moves the pointer **2302** to the next row on the truth table (FIGS. **23A-23B**) and the above procedure of setting of states of all six switches and biopotential data acquisition is repeated. A new data sampling period begins when the pointer **2302** resets (reaches configuration # 1 row) and ends when the pointer **2302** reaches configuration # 64 row. In this manner, left and right side biopotential data is continuously acquired by the smart bands **102, 106** for all 64 switch configurations for every data sampling period.

In one example, the rapid switching described above can be undertaken by the primary smart band microcontroller **2216** by utilizing its clock signal. In another example, a field programmable gate array (FPGA) module working in conjunction with the primary smart band microcontroller **2216** can be employed to accomplish the rapid switching.

With reference to FIG. **22** and FIGS. **23A-23B,** for each switch configuration, the right-side biopotential signal ($R_i$) measured by the secondary smart band electrodes **312, 320, 314, 322, 316,** and **324** is acquired by the microcontroller **2210** via an analog-to-digital (A/D) converter **2212.** Using the radio transceiver **2212** and antenna **2214,** the microcontroller **2210** wirelessly sends the right-side biopotential signal ($R_i$) to the primary smart band **102** attached to the user's left wrist. The primary smart band microcontroller **2216** wirelessly receives the right-side biopotential signal ($R_i$) via its radio transceiver **2218** and antenna **2220.** At the same time, the left-side biopotential signal ($L_i$) measured by the primary smart band electrodes **216, 222, 218, 224, 220, 226** is also acquired by the primary smart band microcontroller **2216** via an A/D converter **2222.** Additionally, the left-side biopotential signal ($L_i$) is fed to the first terminal of a differential amplifier **2224** inside the primary smart band **102.** Moreover, the right-side biopotential signal ($R_i$) from the primary smart band microcontroller **2216** is fed via a D/A converter **2226** to the second terminal of the differential amplifier **2224.** The differential amplifier **2224** output ($V_i$) is then acquired by the primary smart band microcontroller **2216** via the A/D converter **2228.**

When biopotential data is acquired via the described switching method as a continuous stream and buffered over a period of time, there will be 64 right-side biopotential signals ($R_i$), 64 left-side biopotential signals ($L_i$), and 64 analog differential signals ($V_i$). The primary smart band microcontroller **2216** can employ a number of techniques to aggregate and combine these 64 biopotential signals to produce higher fidelity biopotential signals. In one example, the 64 right-side biopotential signals ($R_i$) can be aggregated by computing their weighted mean using weights $W_{Ri}$ as per equation 8, whereby total switch configurations $c = 64$:

$$V_R = \frac{\sum_{i=0}^{c-1} W_{Ri} R_i}{\sum_{i=0}^{c-1} W_{Ri}} \qquad (8)$$

Similarly, the 64 left-side biopotential signals ($L_i$) can be aggregated by computing their weighted mean using weights $W_{Li}$ *as* per equation 9, whereby total switch configurations c = *64:*

$$V_L = \frac{\sum_{i=0}^{c-1} W_{Li} L_i}{\sum_{i=0}^{c-1} W_{Li}} \qquad (9)$$

Finally, the 64 analog differential signals ($V_i$) can be aggregated by computing their weighted mean using weights $W_{Vi}$ as per equation 10, whereby total switch configurations c = *64:*

$$V_{Diff} = \frac{\sum_{i=0}^{c-1} W_{Vi} V_i}{\sum_{i=0}^{c-1} W_{Vi}} \qquad (10)$$

In another embodiment, $R_i$, $L_i$, and $V_i$ can be aggregated by employing a log product as per equations 11-13, whereby total switch configurations c = *64:*

$$V_R = log\left(\prod_{i=0}^{c-1} R_i\right) \qquad (11)$$

$$V_L = log\left(\prod_{i=0}^{c-1} L_i\right) \qquad (12)$$

$$V_{Diff} = log\left(\prod_{i=0}^{c-1} V_i\right) \qquad (13)$$

With reference to FIG. 22 and aggregation equations 8-13, the primary smart band microcontroller **2216** can employ various DSP techniques on the biopotential signals $V_R$ and $V_L$; to produce a high-fidelity ECG signal. In one example, a single-lead ECG signal ($ECG_{Digital}$) is synthesized by the primary smart band microcontroller **2216** by computing the difference between the biopotential signals $V_R$ and $V_L$ as per equation 14:

$$ECG_{Digital} = (V_L - V_R) \qquad (14)$$

In another example, a single-lead ECG signal ($ECG_{Digital}$) is synthesized by the primary smart band microcontroller **2216** by computing the weighted mean of the biopotential signals $V_R$ and $V_L$ using respective weights $W_R$ and $W_L$ as per equation 15:

$$ECG_{Digital} = \frac{(W_L V_L + W_R V_R)}{(W_L + W_R)} \qquad (15)$$

In yet another example, a single-lead ECG signal ($ECG_{Digital}$) is synthesized by the primary smart band microcontroller 2216 by computing a convolution between the biopotential signals $V_R$ and $V_L$ as per equation 16, whereby n is the number of samples in the $V_R$ and $V_L$ arrays:

$$ECG_{Digital}[n] = V_R[n] * V_L[n] = \sum_{k=-\infty}^{\infty} V_R[k].V_L[n-k] \qquad (16)$$

As per FIG. **22** and aggregation equations 8-13, the primary smart band microcontroller **2216** also synthesizes the signal $V_{Diff}$, which is the result of the analog signal amplification and conditioning of $R_i$ and $L_i$ via the differential amplifier **2224**. Therefore, the high-fidelity analog ECG signal *($ECG_{Analog}$),* can be defined via equation 17 as follows:

$$ECG_{Analog} = V_{Diff} \qquad (17)$$

$ECG_{Digital}$ (equations (14)-(16)) and $ECG_{Analog}$ (equation (17)) represent high-fidelity ECG signals that are obtained via two very distinct and complementary techniques - DSP and analog signal conditioning respectively.

In one example, the primary smart band microcontroller **2216** (FIG. **22)** combines and fuses the $ECG_{Digital}$ and $ECG_{Analog}$ signals to further suppress noise and obtain an even higher quality and fidelity signal, namely, $ECG_{Fusion}$ as per equation 18:

$$ECG_{Fusion} = \sqrt{\left(ECG_{Digital}\right)^2 + \left(ECG_{Analog}\right)^2} \qquad (18)$$

There are several other ways by which $ECG_{Fusion}$ can be computed. In one example, $ECG_{Fusion}$ is computed as an arithmetic mean of $ECG_{Digital}$ and $ECG_{Analog}$ as per equation (19):

$$ECG_{Fusion} = \frac{ECG_{Digital} + ECG_{Analog}}{2} \qquad (19)$$

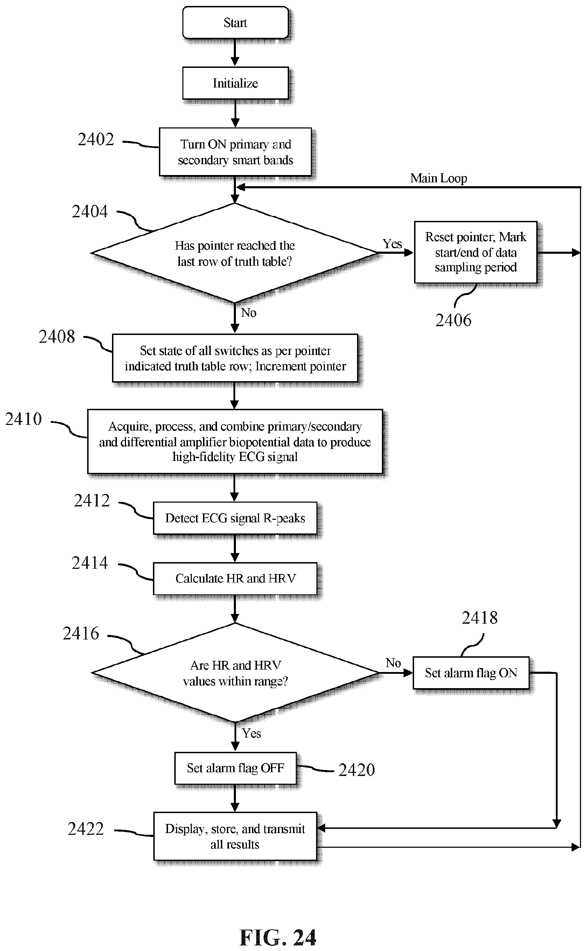

FIG. **24** illustrates a flowchart depicting one example method of continuous high-fidelity ECG monitoring and HR/HRV analysis via electrode switching. At step **2402** both primary **102** and secondary **106** smart bands are switched on using buttons **210** and **310**. At step **2404** the microcontroller **2216** inside the primary smart band checks whether the pointer **2302** has reached the end of the truth table stored inside the primary smart band memory **2230.** If pointer **2302** has not reached the end of the truth table at step **2404,** then at step **2408,** the states of all six switches are set as per the row indicated by the pointer, biopotential data acquisition is initiated, and the pointer is incremented by 1. However, if pointer **2302** has reached the end of the truth table at step **2404,** then, at step **2406,** the pointer is reset, and start/end of a sampling period is marked. The above procedure continues in the main loop for all pointer positions and resets. At step **2410,** the primary smart band microcontroller **2216** performs various operations on the received biopotential data like processing, aggregation, and fusion to produce high-fidelity ECG waveform data. At step **2412,** the primary smart band microcontroller **2216** detects ECG R-peaks and then at step **2414** it computes metrics like HR and HRV. In this example, at step **2416,** the primary smart band microcontroller **2216** checks the calculated HR/HRV metrics against predefined acceptable values. Based on whether the calculated HR/HRV parameters are in range or out of range, alarm flags are accordingly set at steps **2418** and **2420.** At step **2422,** the primary smart band touchscreen display **204** displays ECG waveform data and related analytics along with the alarm status in real-time. Moreover, at step **2422,** the primary smart band wirelessly transmits all ECG waveform data and related analytics to third-party devices **110.**

It will be appreciated by one skilled in the art that variants can exist in the above-described arrangements and applications.

For example, in one embodiment, the described smart band pair can also be used for intermittent ECG monitoring and analysis. For example, the user can operate the on/off switches **210, 310** on the primary and secondary smart bands **102, 106** to enable and disable ECG waveform data acquisition and analysis as required. In another example, the microcontrollers **910, 916,** inside the primary and secondary smart bands **102, 106** can be programmed to acquire and analyze ECG waveform data at predefined intervals, for example, acquire and analyze ECG waveform data for 5 minutes every 30 minutes.

In another embodiment, the described smart band pair can be used solely for biopotential data acquisition and transmission while all data processing/analysis can be done on external devices. For example, the primary smart band **102** can acquire and wirelessly transmit the first biopotential data to a smartphone and the secondary smart band **106** can acquire and wirelessly transmit the second biopotential data to the same smartphone. This smartphone can then process and combine the received first and second biopotential data to produce a high-fidelity ECG signal. The smartphone can also perform further analyses on the ECG signal like R-peak detection, HR/HRV evaluation, and alarm generation. The said smartphone can be replaced by a laptop, tablet, and/or any similar computing device.

In addition, while certain functions have been attributed to the primary smart band for the purpose of illustration in the foregoing description, it will be appreciated that the functions and abilities of the smart bands can be interchanged and/or shared between the primary and secondary smart bands.

**Claims**

**1.** An electrocardiogram monitor comprising:

(a) a primary smart band (102) having a primary microcontroller (914), at least three electrodes, and at least one digital switch per electrode to enable or disable the electrode during data acquisition, wherein the at least three electrodes are configured to contact skin of a user and measure a first high-fidelity biopotential signal;

(b) a secondary smart band (106) having a secondary microcontroller (906), at least three electrodes, and at least one digital switch per electrode to enable or disable the electrode during data acquisition, wherein the at least three electrodes are configured to contact the skin of the user and measure a second high-fidelity biopotential signal;

(c) wherein the primary and secondary microcontrollers (914, 906) control the digital switches to repeatedly loop through all possible configurations of the digital switches and acquire first and second high-fidelity biopotential signals for each of the configurations;

(d) wherein, for each of the configurations of the digital switches, the secondary microcontroller (906) digitizes the second high-fidelity biopotential signal to produce a second digitized signal and transmits the second digitized signal wirelessly to the primary smart band (102);

(e) wherein, for each of the configurations of the digital switches, the primary microcontroller (914) wirelessly receives the second digitized signal from the secondary smart band (106), and digitizes the first high-fidelity biopotential signal to produce a first digitized signal;

(f) wherein the primary microcontroller (914) aggregates the first and second digitized signals for all of the switch configurations and employs DSP techniques on the aggregated first and second digitized signals to produce a first high-fidelity ECG waveform signal;

(g) wherein the primary smart band further comprises a D/A converter (1102) to convert the second digitized signal to an analog signal; and a differential amplifier (1104) which, for each of the configurations of the digital switches, receives as inputs the analog signal from the D/A converter (1102) and the first high-fidelity biopotential signal and outputs a high-fidelity differential signal via analog signal conditioning and amplification;

(h) wherein the primary microcontroller (914) digitizes and aggregates the high-fidelity differential signal for all of the switch configurations to produce a second high-fidelity ECG waveform signal; and

(i) wherein the primary microcontroller (906) employs data fusion techniques to combine the first and second high-fidelity ECG waveform signals to produce a higher quality and fidelity ECG waveform signal.

2. The electrocardiogram monitor of claim 1 wherein at least one of the three electrodes of the primary and secondary smart bands include a reference electrode and the digital switches for the reference electrodes are changeover switches that allow these reference electrodes to be used either as RLD or ground electrodes during data acquisition to further improve ECG waveform signal quality and fidelity.

3. The electrocardiogram monitor of claim 1 or 2 wherein the primary and secondary smart bands each further comprise:

an enclosure (202) having a backplate (228, 318); and
straps (206, 208, 306, 308) connected to the enclosure (202),
wherein the at least three electrodes of the primary and secondary smart bands further comprise:
at least three rigid strip electrodes (216, 218, 220, 312, 314, 316) provided on each of the backplates of the primary and secondary smart bands; and
at least three flexible strip electrodes (222, 224, 226, 320, 322, 324) provided on each of the straps;
wherein the at least three rigid strip electrodes are electrically connected to respective electrodes of the at least three flexible strip electrodes to maximize electrode contact area and eliminate dependency on electrode position around a limb of the user to enhance ECG waveform signal quality.

4. The electrocardiogram monitor of any one of claims 1 to 3 further comprising data storage for storing the first and second ECG signals and related information, and wherein the primary smart band and secondary smart band optionally comprise separate power sources.

5. The electrocardiogram monitor of any one of claims 1 to 4 further comprising a radio transceiver (910, 916) and antenna (912, 918) in the primary smart band or the secondary smart band for transmitting the higher quality and fidelity ECG waveform signal and related information to a separate computing device selected from the group consisting of a mobile device, smartphone, tablet, laptop, and computer.

6. The electrocardiogram monitor of any one of claims 1 to 5 further comprising a display 204 configured to display information to the user, wherein the information is selected from one or more of the group consisting of time, date, battery strength, wireless connectivity strength, Bluetooth status, HR, HRV, ECG waveform and alarm status, and wherein the display is optionally a touchscreen display that is configured to receive inputs from the user; and/or further comprising an alarm, wherein the first microcontroller computes HR and HRV data and triggers and displays the alarm if the HR and/or HRV data are beyond pre-determined thresholds.

7. The electrocardiogram monitor of any one of claims 1 to 6 further comprising a twin wireless charger (802) for charging the primary smart band and secondary smart band.

8. The electrocardiogram monitor of any one of claims 1 to 7 wherein the primary and secondary smart bands are configured to be attached at various locations along limbs of the user.

**Patentansprüche**

1. Elektrokardiogramm-Monitor, umfassend:

(a) ein primäres Smart-Armband (102) mit einem primären Mikrocontroller (914), mindestens drei Elektroden und mindestens einem digitalen Schalter pro Elektrode, um die Elektrode während der Datenerfassung zu aktivieren oder zu deaktivieren, wobei die mindestens drei Elektroden konfiguriert sind, um die Haut eines Benutzers zu berühren und ein erstes hochgenaues Biopotenzialsignal zu messen;

(b) ein sekundäres Smart-Armband (106) mit einem sekundären Mikrocontroller (906), mindestens drei Elektroden und mindestens einem digitalen Schalter pro Elektrode, um die Elektrode während der Datenerfassung zu aktivieren oder zu deaktivieren, wobei die mindestens drei Elektroden konfiguriert sind, um die Haut des Benutzers zu berühren und ein zweites hochgenaues Biopotenzialsignal zu messen;

(c) wobei die primären und sekundären Mikrocontroller (914, 906) die digitalen Schalter steuern, um alle möglichen Konfigurationen der digitalen Schalter wiederholt zu durchlaufen und für jede der Konfigurationen erste und zweite hochgenaue Biopotenzialsignale zu erfassen;

(d) wobei für jede der Konfigurationen der digitalen Schalter der sekundäre Mikrocontroller (906) das zweite hochgenaue Biopotenzialsignal digitalisiert, um ein zweites digitalisiertes Signal zu erzeugen, und das zweite digitalisierte Signal drahtlos an das primäre Smart-Armband (102) überträgt;

(e) wobei für jede der Konfigurationen der digitalen Schalter der primäre Mikrocontroller (914) das zweite digitalisierte Signal drahtlos von dem sekundären Smart-Armband (106) empfängt und das erste hochgenaue Biopotenzialsignal digitalisiert, um ein erstes digitalisiertes Signal zu erzeugen;

(f) wobei der primäre Mikrocontroller (914) die ersten und zweiten digitalisierten Signale für alle Schalterkonfigurationen aggregiert und DSP-Techniken auf die aggregierten ersten und zweiten digitalisierten Signale anwendet, um ein erstes hochgenaues EKG-Wellenformsignal zu erzeugen;

(g) wobei das primäre Smart-Armband ferner einen D/A-Wandler (1102) zum Umwandeln des zweiten digitalisierten Signals in ein analoges Signal; und einen Differenzverstärker (1104) umfasst, der für jede der Konfigurationen der digitalen Schalter als Eingänge das analoge Signal von dem D/A-Wandler (1102) und das erste hochgenaue Biopotenzialsignal empfängt und über analoge Signalkonditionierung und -verstärkung ein hochgenaues Differenzsignal ausgibt;

(h) wobei der primäre Mikrocontroller (914) das hochgenaue Differenzsignal für alle Schalterkonfigurationen digitalisiert und aggregiert, um ein zweites hochgenaues EKG-Wellenformsignal zu erzeugen; und

(i) wobei der primäre Mikrocontroller (906) Datenfusionstechniken anwendet, um die ersten und zweiten hochgenauen EKG-Wellenformsignale zu kombinieren, um ein EKG-Wellenformsignal höherer Qualität und Wiedergabetreue zu erzeugen.

2. Elektrokardiogramm-Monitor nach Anspruch 1, wobei mindestens eine der drei Elektroden der primären und sekundären Smart-Armbänder eine Referenzelektrode beinhaltet und die digitalen Schalter für die Referenzelektroden Umschalter sind, die es ermöglichen, diese Referenzelektroden während der Datenerfassung entweder als RLD- oder als Masseelektroden zu verwenden, um die Qualität und Wiedergabetreue des EKG-Wellenformsignals weiter zu verbessern.

3. Elektrokardiogramm-Monitor nach Anspruch 1 oder 2, wobei die primären und sekundären Smart-Armbänder jeweils ferner umfassen:
ein Gehäuse (202) mit einer Rückplatte (228, 318); und

Bänder (206, 208, 306, 308), die mit dem Gehäuse (202) verbunden sind,
wobei die mindestens drei Elektroden der primären und sekundären Smart-Armbänder ferner umfassen:

mindestens drei starre Streifenelektroden (216, 218, 220, 312, 314, 316), die auf jeder der Rückplatten der primären und sekundären Smart-Armbänder vorgesehen sind; und

mindestens drei flexible Streifenelektroden (222, 224, 226, 320, 322, 324), die auf jedem der Bänder vorgesehen sind;

wobei die mindestens drei starren Streifenelektroden elektrisch mit jeweiligen Elektroden der mindestens drei flexiblen Streifenelektroden verbunden sind, um die Elektrodenkontaktfläche zu maximieren und die Abhängigkeit von der Elektrodenposition um eine Gliedmaße des Benutzers zu eliminieren, um die Qualität des EKG-Wellenformsignals zu verbessern.

4. Elektrokardiogramm-Monitor nach einem der Ansprüche 1 bis 3, ferner umfassend einen Datenspeicher zum Speichern der ersten und zweiten EKG-Signale und zugehöriger Informationen, und wobei das primäre Smart-Armband und das sekundäre Smart-Armband optional separate Stromquellen umfassen.

5. Elektrokardiogramm-Monitor nach einem der Ansprüche 1 bis 4, ferner umfassend einen Funk-Sendeempfänger (910, 916) und eine Antenne (912, 918) in dem primären Smart-Armband oder dem sekundären Smart-Armband zum Übertragen des EKG-Wellenformsignals höherer Qualität und Wiedergabetreue und zugehöriger Informationen an eine separate Rechenvorrichtung, die aus der Gruppe bestehend aus einem mobilen Gerät, Smartphone, Tablet, Laptop und Computer ausgewählt ist.

6. Elektrokardiogramm-Monitor nach einem der Ansprüche 1 bis 5, ferner umfassend ein Display (204), das konfiguriert ist, um dem Benutzer Informationen anzuzeigen, wobei die Informationen aus einem oder mehreren der Gruppe bestehend aus Uhrzeit, Datum, Akkustärke, Stärke der drahtlosen Konnektivität, Bluetooth-Status, HF, HFV, EKG-Wellenform und Alarmstatus ausgewählt sind, und wobei das Display optional ein Touchscreen-Display ist, das konfiguriert ist, um Eingaben von dem Benutzer zu empfangen; und/oder ferner umfassend einen Alarm, wobei der erste Mikrocontroller HF- und HFV-Daten berechnet und den Alarm auslöst und anzeigt, wenn die HF- und/oder HFV-Daten außerhalb vorbestimmter Schwellenwerte liegen.

7. Elektrokardiogramm-Monitor nach einem der Ansprüche 1 bis 6, ferner umfassend ein drahtloses Doppelladegerät (802) zum Aufladen des primären Smart-Armbands und des sekundären Smart-Armbands.

8. Elektrokardiogramm-Monitor nach einem der Ansprüche 1 bis 7, wobei die primären und sekundären Smart-Armbänder konfiguriert sind, um an verschiedenen Stellen entlang der Gliedmaßen des Benutzers angebracht zu werden.

**Revendications**

1. Un moniteur d'électrocardiogramme, comprenant :

(a) un bracelet intelligent principal (102) ayant un microcontrôleur principal (914), au moins trois électrodes, et au moins un commutateur numérique par électrode pour activer ou désactiver l'électrode pendant l'acquisition de données, dans lequel les au moins trois électrodes sont configurées pour entrer en contact avec la peau d'un utilisateur et mesurer un premier signal de biopotentiel à haute fidélité ;

(b) un bracelet intelligent secondaire (106) ayant un microcontrôleur secondaire (906), au moins trois électrodes, et au moins un commutateur numérique par électrode pour activer ou désactiver l'électrode pendant l'acquisition de données, dans lequel les au moins trois électrodes sont configurées pour entrer en contact avec la peau de l'utilisateur et mesurer un second signal de biopotentiel à haute fidélité ;

(c) dans lequel les microcontrôleurs principal et secondaire (914, 906) commandent les commutateurs numériques pour parcourir de manière répétée toutes les configurations possibles des commutateurs numériques et acquérir des premier et second signaux de biopotentiel à haute fidélité pour chacune des configurations ;

(d) dans lequel, pour chacune des configurations des commutateurs numériques, le microcontrôleur secondaire (906) numérise le second signal de biopotentiel à haute fidélité pour produire un second signal numérisé et transmet le second signal numérisé sans fil au bracelet intelligent principal (102) ;

(e) dans lequel, pour chacune des configurations des commutateurs numériques, le microcontrôleur principal (914) reçoit sans fil le second signal numérisé depuis le bracelet intelligent secondaire (106), et numérise le premier signal de biopotentiel à haute fidélité pour produire un premier signal numérisé ;

(f) dans lequel le microcontrôleur principal (914) agrège les premier et second signaux numérisés pour toutes les configurations de commutateur et emploie des techniques de DSP sur les premier et second signaux numérisés agrégés pour produire un premier signal de forme d'onde d'ECG à haute fidélité ;

(g) dans lequel le bracelet intelligent principal comprend en outre un convertisseur N/A (1102) pour convertir le second signal numérisé en un signal analogique ; et un amplificateur différentiel (1104) qui, pour chacune des configurations des commutateurs numériques, reçoit en tant qu'entrées le signal analogique provenant du convertisseur N/A (1102) et le premier signal de biopotentiel à haute fidélité et délivre en sortie un signal différentiel à haute fidélité via un conditionnement et une amplification de signal analogique ;

(h) dans lequel le microcontrôleur principal (914) numérise et agrège le signal différentiel à haute fidélité pour toutes les configurations de commutateur pour produire un second signal de forme d'onde d'ECG à haute fidélité ; et

(i) dans lequel le microcontrôleur principal (906) emploie des techniques de fusion de données pour combiner les premier et second signaux de forme d'onde d'ECG à haute fidélité pour produire un signal de forme d'onde d'ECG de qualité et de fidélité supérieures.

2. Le moniteur d'électrocardiogramme selon la revendication 1, dans lequel au moins une des trois électrodes des bracelets intelligents principal et secondaire inclut une électrode de référence et les commutateurs numériques pour les électrodes de référence sont des commutateurs d'inversion qui permettent à ces électrodes de référence d'être utilisées soit comme électrodes RLD, soit comme électrodes de masse pendant l'acquisition de données pour améliorer davantage la qualité et la fidélité du signal de forme d'onde d'ECG.

3. Le moniteur d'électrocardiogramme selon la revendication 1 ou 2, dans lequel les bracelets intelligents principal et secondaire comprennent en outre chacun :

   un boîtier (202) ayant une plaque arrière (228, 318) ; et
   des sangles (206, 208, 306, 308) connectées au boîtier (202),
   dans lequel les au moins trois électrodes des bracelets intelligents principal et secondaire comprennent en outre :

      au moins trois électrodes en bande rigides (216, 218, 220, 312, 314, 316) prévues sur chacune des plaques arrière des bracelets intelligents principal et secondaire ; et
      au moins trois électrodes en bande flexibles (222, 224, 226, 320, 322, 324) prévues sur chacune des sangles ;
      dans lequel les au moins trois électrodes en bande rigides sont connectées électriquement à des électrodes respectives des au moins trois électrodes en bande flexibles pour maximiser la surface de contact d'électrode et éliminer la dépendance de la position de l'électrode autour d'un membre de l'utilisateur pour améliorer la qualité du signal de forme d'onde d'ECG.

4. Le moniteur d'électrocardiogramme selon l'une quelconque des revendications 1 à 3, comprenant en outre une mémoire de données pour stocker les premier et second signaux d'ECG et des informations associées, et dans lequel le bracelet intelligent principal et le bracelet intelligent secondaire comprennent optionnellement des sources d'alimentation séparées.

5. Le moniteur d'électrocardiogramme selon l'une quelconque des revendications 1 à 4, comprenant en outre un émetteur-récepteur radio (910, 916) et une antenne (912, 918) dans le bracelet intelligent principal ou le bracelet intelligent secondaire pour transmettre le signal de forme d'onde d'ECG de qualité et de fidélité supérieures et des informations associées à un dispositif informatique séparé sélectionné dans le groupe constitué d'un dispositif mobile, d'un smartphone, d'une tablette, d'un ordinateur portable et d'un ordinateur.

6. Le moniteur d'électrocardiogramme selon l'une quelconque des revendications 1 à 5, comprenant en outre un afficheur (204) configuré pour afficher des informations à l'utilisateur, dans lequel les informations sont sélectionnées parmi un ou plusieurs du groupe constitué de l'heure, la date, le niveau de la batterie, l'intensité de la connectivité sans fil, l'état Bluetooth, la FC, la VFC, la forme d'onde d'ECG et l'état d'alarme, et dans lequel l'afficheur est optionnellement un afficheur à écran tactile qui est configuré pour recevoir des entrées de l'utilisateur ; et/ou comprenant en outre une alarme, dans lequel le premier microcontrôleur calcule des données de FC et de VFC et déclenche et affiche l'alarme si les données de FC et/ou de VFC sont au-delà de seuils prédéterminés.

7. Le moniteur d'électrocardiogramme selon l'une quelconque des revendications 1 à 6, comprenant en outre un chargeur double sans fil (802) pour charger le bracelet intelligent principal et le bracelet intelligent secondaire.

8. Le moniteur d'électrocardiogramme selon l'une quelconque des revendications 1 à 7, dans lequel les bracelets intelligents principal et secondaire sont configurés pour être fixés à divers emplacements le long des membres de l'utilisateur.

**FIG. 1**

FIG. 2A

FIG. 2B

FIG. 2C

**FIG. 3A**

**FIG. 3B**

**FIG. 3C**

EP 3 934 527 B1

FIG. 4

FIG. 5

**FIG. 7**

**FIG. 6**

FIG. 8

FIG. 9

FIG. 10

**FIG. 11**

**FIG. 12**

**FIG. 13**

**FIG. 14**

Primary Smart Band

Secondary Smart Band

**FIG. 15**

Start

↓

Initialize

↓

1602 — Turn ON primary and secondary smart bands

Main Loop

↓

1604 — Is biopotential data received from primary and secondary smart bands valid?  → No → Sleep

↓ Yes

1606 — Is primary and secondary smart band biopotential data ready?  → No →

↓ Yes

1608 — Apply digital filtering, process, and combine primary/secondary smart band biopotential data to produce high-fidelity ECG signal

↓

1610 — Detect ECG signal R-peaks

↓

1612 — Calculate HR and HRV

↓

1614 — Are HR and HRV values within range?  → No → 1616 Set alarm flag ON

↓ Yes

Set alarm flag OFF — 1618

↓

1620 — Display, store, and transmit all results

**FIG. 16**

**FIG. 17**

**FIG. 18**

**FIG. 19**

$$ECG_{Fusion} = \sqrt{(ECG_{Digital})^2 + (ECG_{Analog})^2}$$

ECG Via DSP ($ECG_{Digital}$)

ECG Via Analog Signal Conditioning ($ECG_{Analog}$)

FIG. 20

Start

↓

Initialize

↓

2102 — Turn ON primary and secondary smart bands

Main Loop

↓

2104 — Is biopotential data from primary/secondary smart bands and differential amplifier valid? —No→ Sleep

↓ Yes

2106 — Is primary/secondary and differential amplifier biopotential data ready? —No→

↓ Yes

2108 — Apply digital filtering, process, and fuse primary/secondary and differential amplifier biopotential data to produce high-fidelity ECG signal

↓

2110 — Detect ECG signal R-peaks

↓

2112 — Calculate HR and HRV

↓

2114 — Are HR and HRV values within range? —No→ 2116 Set alarm flag ON

↓ Yes

Set alarm flag OFF — 2118

↓

2120 — Display, store, and transmit all results

**FIG. 21**

40

**FIG. 22**

Pointer
*Start*

2302

| # | Secondary Smart Band | | | Primary Smart Band | | |
|---|---|---|---|---|---|---|
| | $S_{LS}$ | $S_{RS}$ | $S_{DS}$ | $S_{LP}$ | $S_{RP}$ | $S_{DP}$ |
| 1 | 0 | 0 | 0 | 0 | 0 | 0 |
| 2 | 0 | 0 | 0 | 0 | 0 | 1 |
| 3 | 0 | 0 | 0 | 0 | 1 | 0 |
| 4 | 0 | 0 | 0 | 0 | 1 | 1 |
| 5 | 0 | 0 | 0 | 1 | 0 | 0 |
| 6 | 0 | 0 | 0 | 1 | 0 | 1 |
| 7 | 0 | 0 | 0 | 1 | 1 | 0 |
| 8 | 0 | 0 | 0 | 1 | 1 | 1 |
| 9 | 0 | 0 | 1 | 0 | 0 | 0 |
| 10 | 0 | 0 | 1 | 0 | 0 | 1 |
| 11 | 0 | 0 | 1 | 0 | 1 | 0 |
| 12 | 0 | 0 | 1 | 0 | 1 | 1 |
| 13 | 0 | 0 | 1 | 1 | 0 | 0 |
| 14 | 0 | 0 | 1 | 1 | 0 | 1 |
| 15 | 0 | 0 | 1 | 1 | 1 | 0 |
| 16 | 0 | 0 | 1 | 1 | 1 | 1 |
| 17 | 0 | 1 | 0 | 0 | 0 | 0 |
| 18 | 0 | 1 | 0 | 0 | 0 | 1 |
| 19 | 0 | 1 | 0 | 0 | 1 | 0 |
| 20 | 0 | 1 | 0 | 0 | 1 | 1 |
| 21 | 0 | 1 | 0 | 1 | 0 | 0 |
| 22 | 0 | 1 | 0 | 1 | 0 | 1 |
| 23 | 0 | 1 | 0 | 1 | 1 | 0 |
| 24 | 0 | 1 | 0 | 1 | 1 | 1 |
| 25 | 0 | 1 | 1 | 0 | 0 | 0 |
| 26 | 0 | 1 | 1 | 0 | 0 | 1 |
| 27 | 0 | 1 | 1 | 0 | 1 | 0 |
| 28 | 0 | 1 | 1 | 0 | 1 | 1 |
| 29 | 0 | 1 | 1 | 1 | 0 | 0 |
| 30 | 0 | 1 | 1 | 1 | 0 | 1 |
| 31 | 0 | 1 | 1 | 1 | 1 | 0 |
| 32 | 0 | 1 | 1 | 1 | 1 | 1 |

**FIG. 23A**

| # | Secondary Smart Band | | | Primary Smart Band | | |
|---|---|---|---|---|---|---|
| | $S_{LS}$ | $S_{RS}$ | $S_{DS}$ | $S_{LP}$ | $S_{RP}$ | $S_{DP}$ |
| 33 | 1 | 0 | 0 | 0 | 0 | 0 |
| 34 | 1 | 0 | 0 | 0 | 0 | 1 |
| 35 | 1 | 0 | 0 | 0 | 1 | 0 |
| 36 | 1 | 0 | 0 | 0 | 1 | 1 |
| 37 | 1 | 0 | 0 | 1 | 0 | 0 |
| 38 | 1 | 0 | 0 | 1 | 0 | 1 |
| 39 | 1 | 0 | 0 | 1 | 1 | 0 |
| 40 | 1 | 0 | 0 | 1 | 1 | 1 |
| 41 | 1 | 0 | 1 | 0 | 0 | 0 |
| 42 | 1 | 0 | 1 | 0 | 0 | 1 |
| 43 | 1 | 0 | 1 | 0 | 1 | 0 |
| 44 | 1 | 0 | 1 | 0 | 1 | 1 |
| 45 | 1 | 0 | 1 | 1 | 0 | 0 |
| 46 | 1 | 0 | 1 | 1 | 0 | 1 |
| 47 | 1 | 0 | 1 | 1 | 1 | 0 |
| 48 | 1 | 0 | 1 | 1 | 1 | 1 |
| 49 | 1 | 1 | 0 | 0 | 0 | 0 |
| 50 | 1 | 1 | 0 | 0 | 0 | 1 |
| 51 | 1 | 1 | 0 | 0 | 1 | 0 |
| 52 | 1 | 1 | 0 | 0 | 1 | 1 |
| 53 | 1 | 1 | 0 | 1 | 0 | 0 |
| 54 | 1 | 1 | 0 | 1 | 0 | 1 |
| 55 | 1 | 1 | 0 | 1 | 1 | 0 |
| 56 | 1 | 1 | 0 | 1 | 1 | 1 |
| 57 | 1 | 1 | 1 | 0 | 0 | 0 |
| 58 | 1 | 1 | 1 | 0 | 0 | 1 |
| 59 | 1 | 1 | 1 | 0 | 1 | 0 |
| 60 | 1 | 1 | 1 | 0 | 1 | 1 |
| 61 | 1 | 1 | 1 | 1 | 0 | 0 |
| 62 | 1 | 1 | 1 | 1 | 0 | 1 |
| 63 | 1 | 1 | 1 | 1 | 1 | 0 |
| 64 | 1 | 1 | 1 | 1 | 1 | 1 |

2302
Pointer
*End*

**FIG. 23B**

42

Start

Initialize

2402 — Turn ON primary and secondary smart bands

Main Loop

2404 — Has pointer reached the last row of truth table?

Yes — Reset pointer; Mark start/end of data sampling period

2406

No

2408 — Set state of all switches as per pointer indicated truth table row; Increment pointer

2410 — Acquire, process, and combine primary/secondary and differential amplifier biopotential data to produce high-fidelity ECG signal

2412 — Detect ECG signal R-peaks

2414 — Calculate HR and HRV

2416 — Are HR and HRV values within range?

No — Set alarm flag ON — 2418

Yes

Set alarm flag OFF — 2420

2422 — Display, store, and transmit all results

**FIG. 24**